# EUROPEAN PATENT APPLICATION

(11) **EP 2 253 331 A1**
(43) Date of publication of application: **24.11.2010**
(21) Application number: 10175865.4
(22) Date of filing: 02.08.2002
(51) Int. Cl.: A61K 48/00, A01K 67/027, G01N 33/50

(54) **Modulation of antibody formation in mammals, applicability in gene therapy and animal model generation**

(30) Priority: 03.08.2001 US 920902
(62) Divisional of application: 02765165.2
(71) Applicant: Nokad, 91000 Evry (FR)
(72) Inventor: Abina, Amine, 92250 La GarenneColombes (FR)
(74) Representative: Warcoin, Jacques

(57) **Abstract**

The invention relates to a method of modulating neutralizing antibodies formation against an heterologous protein. The invention also relates to the use of such method to induce a tolerisation of the immune system towards said protein, such tolerisation being useful to allow long-term gene therapy or transgene expression. The invention also relates to the use of the method of the invention to provide an animal with a reproducible functional inactivation phenotype of an endogenous protein of the animal.

## Description

The present invention relates to the field of biology, more precisely to the field of animal transgenesis and somatic gene therapy and methods useful therein. The invention relates to a method for modulating the capacity of a mammal to produce neutralizing antibodies against one or more immunogenic material(s) administered to said mammal, and the applications of such method to gene therapy, animal somatic transgenesis, animal models having a functional knock-out phenotype.

The introduction of a biologically active protein or a transgene expressing such protein, to a mammalian host cell can have significant therapeutic or economical values. However, this approach also has several drawbacks.

In gene therapy, beside the risk of potential toxicity, the clinical impact of such protein is limited by their relative short half-life and biological activity *in vivo* which results from an induction of a cell-mediated immune response against infected cells. In particular, cytotoxic T lymphocytes (CTLs) have been detected against antigenically expressed viral proteins encoded by viral vectors, such as adenoviral vectors, even though such vectors are replication defective. CTLs have also been detected against immunogenic transgene products. Cytotoxic T lymphocytes have the potential destroy or damage cells harbouring the viral vectors, thereby causing loss of transgene expression. Cell destruction can also cause inflammation which is also detrimental to the tissues involved. The cell-mediated immune response can pose a potentially serious obstacle to therapies requiring high dosages or repeated administration or to production of a recombinant product by a transgenic animal which are likely to elicit more potent immune responses (See Kaplan *et al.,* 1997; Yang *et al.,* 1994, Yang *et al.,* 1996).

In order to circumvent the host immune response which limits the persistence of transgene expression either to human gene therapy or animal transgenesis, various strategies have been employed generally involving either the modulation of the immune response itself, or the engineering of a transgene vector that decreases the immune response. Indeed, in gene therapy, the administration of immunosuppressive agents together with vector administration has been shown to prolong transgene persistence (Fang *et al.,* 1995; Kay *et al.,* 1995; Zsellenger *et al.,* 1995). In another approach, modification of viral genome sequences in recombinant vectors, such as adenoviral vectors, has been used in attempts to decrease recognition of the vector by the immune system (see Yang *et al., 1*994); Lieber *et al.*, 1996) ; Gorziglia *et al.,* 1996) ; Kochanek *et al.,* 1996) ; Fisher *et al.,* 1996)). Additionally, it has been demonstrated that the choice of promoter or transgene may also influence persistence of transgene expression from viral vectors (see *e.g.,* Guo *et al*., 1996; Tripathy *et al.*, 1996).

However, such different approaches have only achieved limited success. Since persistent transgene expression is highly desirable in animal transgenesis and in gene therapy settings, especially those seeking to alleviate chronic or hereditary diseases in mammals, the current state of vector-based gene delivery or transgenesis requires the development of transgene expression systems and methods which demonstrate the capability for persistence and sustained expression of a transgene.

The present invention adresses all of these needs.

The present invention provides a solution to the aforementioned need in the art by providing a method of modulating in a mammal formation of neutralizing antibodies directed against an heterologous protein. The method of the invention allows to determine the amount of an agent sufficient to selectively tolerize a mammalian subject to an heterologous protein, thus eliminating the immune barrier impeding long-term gene therapy or transgene expression. Alternatively, the method of the invention allows to determine the amount of an agent necessary and sufficient to induce in a reproducible way an immune response against the transgene product to generate a functional inactivation of an endogenous protein phenotype.

The details of the preferred embodiment of the present invention are set forth in the accompanying drawings and the description below. Once the details of the invention are known, numerous additional innovations and changes will become obvious to one skilled in the art.

By the term "neutralizing antibodies" as used herein is meant antibodies or a fragment thereof that are able to target the heterologous protein of the invention and hamper its biological activity.

The terms "nucleic acid sequence", "transgene", "gene", "vector" are used herein with the same meaning. Depending of the embodiments of the invention, the nucleic acid sequence encoding said heterologous protein, also named transgene, or gene, is either part of a cloning and/or expression vector, or part of a wild type or recombinant genome of a virus, parasite, fungus, bacteria.

By the term "transgene" as used herein is meant a DNA segment encoding a protein which is partly or entirely heterologous (*i.e*. foreign) to the mammalian host genome. The transgene can be a therapeutic transgene that supplies (whole or in part) a necessary gene product that is totally or partially absent from a mammalian cell or tissue of interest. The transgene can be a transgene encoding for an economical valuable product (*i.e*. usually a therapeutical product) that allows the host to produce such a product from its cells or organ(s). The transgene can be a transgene encoding for a protein having substantial identity to an endogenous protein so as the host to produce neutralizing antibodies against said foreign protein that cross reacts with said endogenous protein, leading to a functional knock-out of said endogenous protein.

As used herein "functional inactivation of an endogenous protein" means the biological inactivation of a protein at the protein level, in opposition with the "conventional knock-out" that it is perform at the gene level by homologous recombination. The neutralizing antibodies directed against an heterologous protein constitute the means to alter the biological activity of the endogenous protein that is substantially identical to the heterologous protein.

"Host" refers to the recipient of the therapy to be practised according to the invention or the recipient in cells of which a transgene is expressed. The host may be a vertebrate, but will preferably be a mammal. If it is a mammal, the host will preferably be a human for the gene therapy applications of the method of the invention but may also be a domestic livestock, pet animal, or a laboratory animal. For the functional inactivation of an endogenous protein, long lasting transgene expression in animal transgenesis, the host will preferably be a mammal, most preferably a laboratory animal, a domestic livestock or a pet animal, but may also be a human. For the functional inactivation of an endogenous protein (*i.e*. functional Knock-out), the mammal is a human, especially in need of a treatment of a disease caused by the expression of an abnormal protein, or a laboratory animal, a domestic livestock or a pet animal.

By "the amount of agent" it means the number of moieties that is administrated to a given mammal. For a virus, this amount includes the recombinant virus particles that encode and express said heterologous protein and incomplete, empty or wild type virus particles that "contaminate" the viral stock.

"Antigen Presenting Cells" or "APC's" include known APC's such as Langerhans cells, veiled cells of afferent lymphatics, dendritic cells and interdigitating cells of lyphoïds organs. The definition also includes mononuclear cells such as lymphocytes and macrophages.

A "vector" is a replicon in which another polynucleotide segment is attached (*i.e*. a transgene), so as to bring the replication and/or expression to the attached segment. Examples of vectors include plasmids, phages, cosmids, phagemid, yeast artificial chromosome (YAC), bacterial artificial chromosome (BAC), human artificial chromosome (HAC), viral vector, such as adenoviral vector, retroviral vector, adeno-associated viral vector and other DNA sequences which are usually able to replicate or to be replicated *in vitro* or in a host cell, or to convey a desired DNA segment to a desired location within a host cell, or to express a desired gene within a host cell, especially APC's cells. Naked DNA molecules, encoding the heterologous protein of the invention, are therefore in the scope of the invention.

A "promoter" or a "promoter sequence" is a DNA regulatory region capable of binding RNA polymerase in a cell and initiating transcription downstream (3'direction) coding sequence. Within the promoter sequence will be found a transcription initiation site, as well as protein binding domains responsible for the binding of RNA polymerase. Eukaryotic promoters will often, but not always, contains TATA boxes and CAT boxes.

A "Tolerigenic Dose" (TD) is a dose of virus injected by an intraveinous route that does not induce a humoral response against the transgene-encoded protein and/or that is neither able to neutralize the long-term biological activity of the transgene-encoded protein nor the biological activity of the endogenous homologous protein.

An "Immunogenic Dose" (ID) is a dose of virus injected by an intraveinous route able to induce a humoral response againt the transgene-encoded protein and is able to neutralize the long-term biological activity of the transgene-encoded protein and/or the biological activity of the endogenous homologous protein.

"Operatively linked" as used herein, includes reference to a functional linkage between a promoter and a second sequence (*i.e*. a nucleic acid sequence of the invention), wherein the promoter sequence initiates and mediates the transcription of said DNA sequence.

"Pharmaceutically acceptable carrier" includes any acceptable solution, dispersion media, coating, antibacterial and antifungal agents, isotonic and absorption delaying agents, and the like.

The invention consists of means of modulating formation of neutralizing antibodies directed to an antigen. Although it is not intented that the invention will be entirely limited by a particular theory as to the mechanism of modulation involved, it is believed that agents of the invention such as viruses (*i.e*. adenoviruses) administrated to a mammal in an amount much greater that the amount sufficient to trigger an immune response, are able to localy saturate or inactivate APC's cells, leading to a tolerization to a compound such as a protein subsequently administrated to said mammal.

The determination of the amount of such agent to induce tolerization toward said protein allows to control the neutralizing antibodies formation in said mammal. This determination will be highly appreciated since it allows either to get the conditions for a sustained and long-lasting expression of a trangene, or the conditions to induce a functional knock-out phenotype when the compound subsequently administrated is a protein homologous to a mammal's endogenous protein.

In prior art, Abina *et al.* (1998) obtained fortuitously a mouse with a thrombopoietin (TPO) knock-out phenotype induced by cross-reactive antibodies against TPO following injection with recombinant adenovirus encoding human TPO. Indeed, the inventor shows in the Examples section (see Results - 2.1) of the present invention that this result varies from an experiment to another (i. e. from a viral stock to another). The inventor of the present invention now ellucidate the biological mechanism and identified the technical effects underlying the results described in Abina *et al.* (1998).

The method of the invention of modulating neutralizing antibodies formation allows either to induce reproductible functional Knock-out phenotype or long-lasting transgene expression. The applications of such method are therefore very important, and constitutes a breakthrough in gene therapy and in the field of the production of animal models. Indeed, in gene therapy such a method allows to circumvent the aforementionned disadvantages of the previous art, and in animal models production, said method allows the generation of an animal model in 3 to 5 months instead of the 1 to 2 years necessary to perform a conventional knock-out animal (*i.e*. a knock-out mouse) by gene targeting or 8-10 months to perform a conventional transgenic animal by pronucleus micro-injection.

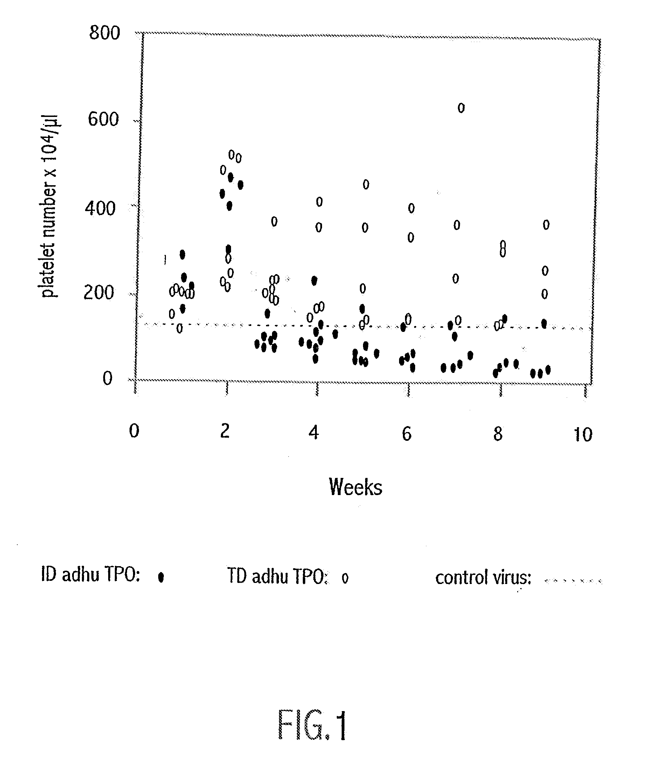

### Figure 1: Differential effect on platelet count of ID versus TD of AdRSVhuTPO. Early thrombocytopenia in the ID-AdRSVhuTPO-injected mice and prolonged thrombocytemia in TD- AdRSVhuTPO-injected mice.

Mice were injected by an immunogenic dose (ID) (2-4 x 10⁹) or a tolerigenic dose (TD) (6-8 x 10⁹) of AdRSVhuTPO (recombinant adenovirus encoding human thrombopoietin gene under the control of the RSV promoter) at day 0 and followed each week for platelet counts in whole blood.

### Figure 2: Example of an immunogenic dose (ID) of AdRSVhuTPO at 6 x 10⁹ pfu by a viral preparation different than the one used for the other experiments.

These results emphasize the importance of determination of the exact dose for the induction of a functional inactivation of an endogenous protein or a long term transgene expression phenotype for each viral preparation.

### Figure 3: Neutralizing activity of mice sera against human and murine TPO in a cell proliferation assay.

HuTPO: human thrombopoietin
MuTPO: murine thrombopoietin

### Figure 4 A, B:

Presence or absence of IgG1 (A) or IgG2a (B) anti-huTPO antibodies depends on the injection of immunogenic dose (ID) or tolerigenic dose (TD) of AdRSVhuTPO respectively. Two representative mice M1 and M2 for each dose are presented for each isotype at week 5 (W5) or at week 13 (W13). OD 492 nm: optic densitometry measured at 492 nm.

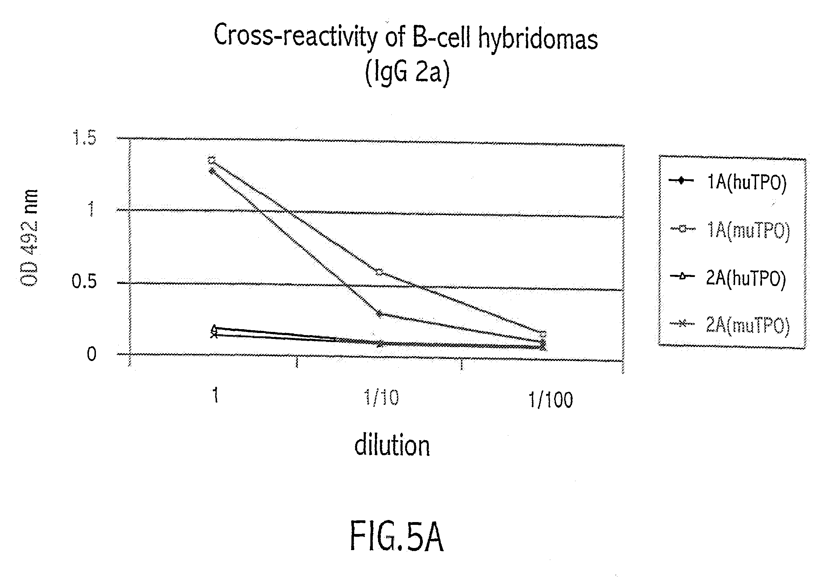

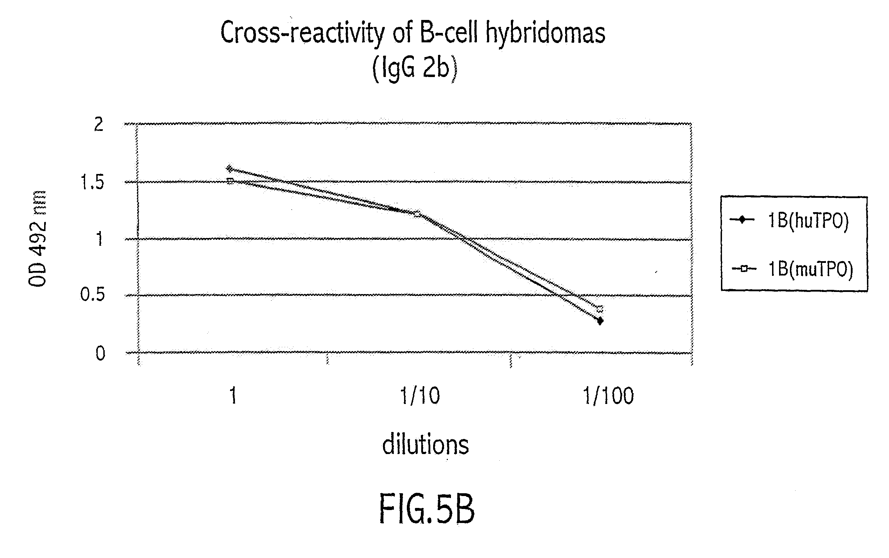

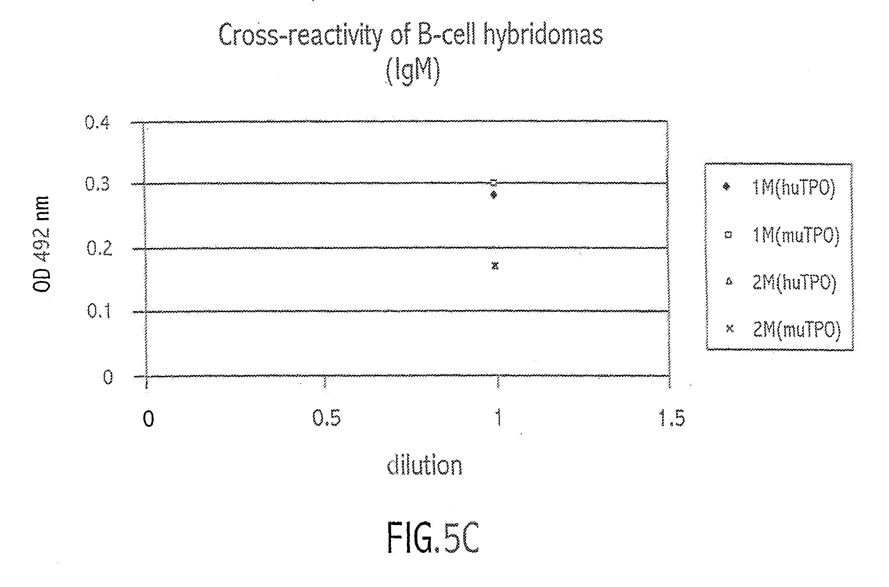

### Figure 5 A, B, C: Cross-reactivity of all monoclonal antibodies derived from thrombocytopenic mice as determined by a classical ELISA test.

IgG2a (A), IgG2b (B) and IgM (C) monoclonal antibodies derived from B-cell hybridomas showed same dilutions profiles in all the cases. Each hybridoma is numbered. The two hydridomas tested expressing IgG2a are numbered 1A, 2A; the hydridoma tested expressing IgG2b is numbered 1B; The two hydridomas tested expressing IgM are numbered 1M, 2M. HuTPO: human thrombopoietin; MuTPO: murine thrombopoietin.

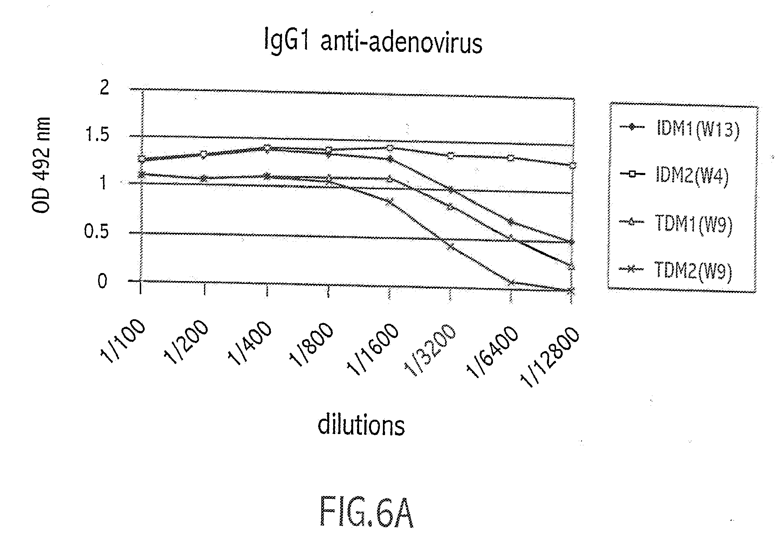

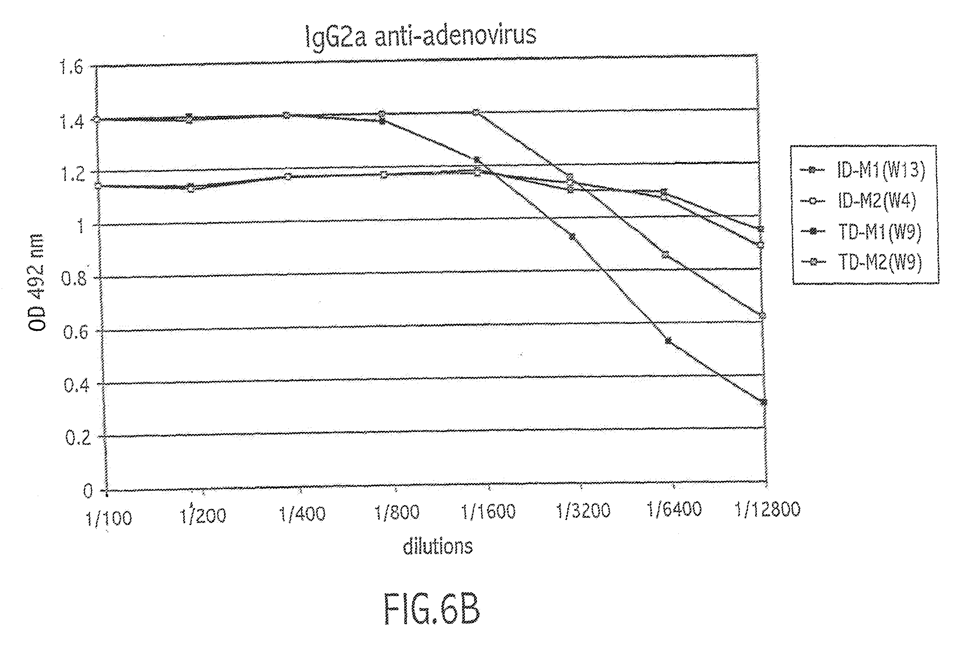

### Figure 6 A, B: Anti-adenovirus antibody detection in the mice sera showed same profiles following an immunogenic dose (ID) injection or a tolerigenic dose (TD) AdRSVhuTPO injection.

An immunogenic dose (ID) or a tolerigenic dose (TD) of AdRSVhuTPO were administratred in mice. IgG1 (A) and IgG2a (B) anti-adenovirus isotypes are detected at different weeks: week 4 (w4), week 9 (w9), week 13 (w13). Two mice M1 and M2 are tested for each dose.

The invention is directed to a method of inhibiting in a mammal formation of neutralizing antibodies directed against an heterologous protein comprising the step of co-administering to said mammal, an agent in an amount sufficient to deplete or inhibit at least some antigen presenting cells (APC) of said mammal, and said heterologous protein and/or a nucleic acid sequence encoding said heterologous protein, said agent being administered prior or simultaneously to said heterologous protein and/or a nucleic acid sequence, thereby inhibiting the production of neutralizing antibodies against said heterologous protein.

Without wishing to be bound by theory, the inventors theorize that the primary stimulus for immune activation is the agent of the invention. This agent that is presented by the APC's is in enough large amount either to deplete or inactivate the antigen presenting activity of the APC's; consequently a subsequent administration of an heterologous protein does not trigger an immune response since no APC's is available to present said protein or fragments thereof, thereby preventing formation of neutralizing antibodies against said heterologous protein.

In the method of the invention, said agent is administered prior said heterologous protein. In a less suitable embodiment, said agent is administered simultaneously with said heterologous protein. Preferably, said agent is administered prior or simultaneously to said nucleic acid sequence encoding said heterologous protein ; indeed, the administration of said nucleic acid sequence cannot trigger an immune response against the heterologous protein before the expression of the latter from said nucleic acid sequence, then when the heterologous protein is expressed, the APC's have been previously saturated or inactivated by the agent previously administered in a large amount. Consequently, when the administration is simultaneous, it is preferred that said agent is administered with a nucleic acid sequence encoding said heterologous protein, in order the first immune response is only directed against the agent. In a preferred embodiment, said agent and said nucleic acid sequence encoding said heterologous protein are simulatenously co-administered as a recombinant virus, the genome of which comprising at least nucleic acid sequence encoding said heterologous protein.

The agent of the invention is selected on its ability to target antigen presenting cells, or to be put into contact with APC's. The agent of the invention is further able to be presented by the APC's. Alternatively, the agent is able to inactivate the APC's, by saturating the APC's cell receptors for example. The agent to be used in the invention is selected among viruses, liposomes, antibodies, parasites, bacteriae, funguses and or fragments thereof, or naked nucleic acid sequence encoding said heterologous protein.

When said agent is a virus, parasites, bacteriae, funguses, the genome of said agent encoding at least for said heterologous protein corresponds either to the wild type genome or has been genetically engineered to encode at least a transgene.

When said agent is a virus, it is preferably selected among adenovirus, adenovirus associated virus, retrovirus, pox virus, vaccinia virus, or fragments thereof. Preferred virus is adenovirus, preferably human adenovirus, that is selected among wild type adenovirus and recombinant adenovirus, or a fragment thereof.

When said agent is a liposome, it preferably contains said nucleic acid sequence encoding said heterologous protein. Such liposomes, which are preferably charged with polysaccharides to allow binding or entry into APC's in a way to inactivate them.

When said agent in a nucleic acid sequence encoding at least for the heterologous protein, said nucleic acid sequence administered to the mammal in a sufficient amount triggers the immune reesponse, and inhibits or depletes the APC's ; the subsequent expression of said heterelogous protein from said nucleic acid sequence does not trigger an immune response, thereby preventing formation of neutralizing antibodies against said heterologous protein.

All agents unable to enter or inactivate the APC's but modified in a way to do so are also in the scope of the invention.

The antigen presenting cells of the invention are any antigen presenting cells of the mammal. Antigen Presenting Cells" or "APC's" include known APC's such as Langerhans cells, veiled cells of afferent lymphatics, dendritic cells and interdigitating cells of lymphoids organs and mononuclear cells such as lymphocytes and macrophages. In a preferred embodiment, the APC's are the ones located in liver of said mammal. In another embodiment, the APC's are the ones located in skin, lung or muscle of said mammal.

The method of the invention may further comprise the step of administering to said mammal additional agent to enhance the depletion and/or the inhibition of at least some antigen presenting cells of said mammal. This additional step can be repeated until the depletion and/or the inhibition of at least some antigen presenting cells of said mammal is reached. This additional agent is preferably a wild-type or recombinant virus, more preferably an adenovirus, the genome of which not containing nor expressing a transgene encoding said heterologous protein. Alternatively, said additional agent may be a viral empty capside, or fragments thereof.

It also may be necessary that the method of the invention further comprises the step(s) of administering to said mammal additional heterologous protein and/or nucleic acid encoding said protein to trigger immune response. Additional administrations can be performed until an immune response is triggered, or until the expression of the heterologous protein by the host cells is sufficient.

In a preferred embodiment, the method of inhibiting in a mammal formation of neutralizing antibodies directed against an heterologous protein comprising the step of administering to said mammal a recombinant adenovirus, the genome of which comprising at least a nucleic acid sequence encoding said heterologous protein and regulation sequences necessary to direct the expression of said heterologous protein in at least one antigen presenting cell of said mammal in an amount sufficient to deplete or inhibit at least some antigen presenting cells of said mammal, thereby inhibiting the production of neutralizing antibodies against said heterologous protein. This method can further comprise the step of administering to said mammal additional adenovirus or a fragment thereof, the genome of which not expressing said heterologous protein, thereby enhancing the amount of adenoviruses to deplete or inhibit at least some antigen presenting cells of said mammal. According to this prefered embodiment, the agent of the invention is a recombinant adenovirus. Recombinant adenovirus have advantages for use as transgene expression systems, including tropism for both dividing and non-dividing cells, minimal pathogenic potential, ability to replicate to high titer for preparation of vector stocks, and the potential to carry large inserts (see *e.g*., Berkner, 1992 ; Jolly, 1994). Adenovirus vectors can accommodate a variety of transgenes of different sizes. For example, about an eight (8) kb insert can be accommodated by deleting regions of the adenovirus genome dispensable for growth (*e.g*., E3). Development of cell lines that supply non-dispensable adenovirus gene products in trans (*e.g*., E1, E2a, E4) allows insertion of a variety of transgenes throughout the adenovirus genome (see *e.g*. Graham, 1977 ; Imler *et al.,* 1996). Preferably the components of the adenovirus transgene expression system (*i.e*. the transcription unit, E3 cassette, E4 cassette) are configured on a single adenovirus vector. Preferably, the adenovirus vector is replication-defective. This is not intended to be limiting of the transgene expression systems, since the components can be configured in a number of ways to meet the intended use. For example, in one preferred embodiment, the adenovirus vector comprises a transcription unit comprising the transgene (i.e the nucleic acid sequence encoding said heterologous protein) inserted into the E1a, E1b region of adenovirus. In this embodiment, the adenovirus vector further comprises the E3 cassette and the E4 cassette configured in positions corresponding to the E3 and E4 regions of adenovirus, respectively. The adenovirus vector largely comprises adenovirus genome sequences, and further comprising at least a portion of an adenovirus E3 region and an E4 ORF3 and at least one portion of E4. Preferably, the adenovirus vector is incapable of productively replicating in host cells unless co-infected with an adenovirus helper virus or introduced into a suitable cell line supplying one or more adenovirus gene products in trans (*e.g*., 293 cells). Adenoviruses with larger deletion of the viral genome (Maione *et al.,* 2001) can also be used for the applications described in the invention. An adenovirus vector according to the invention can belong to any one of the known six human subgroups, *e.g*., A, B, C, D, E, or F, wherein a preferred series of serotypes (all from subgroup D) includes Ad9, Ad15, Ad17, Ad19, Ad20, Ad22, Ad26, Ad27, Ad28, Ad30, or Ad39. Preferred serotypes include the Ad2 and Ad5 serotypes. Additionally, chimeric adenovirus vectors comprising combinations of Ad DNA from different serotypes are within the scope of the present invention. Adenoviruses from other species (porcine, ovine, bovine, canine, murine etc...) can also be used for the same purpose. The adenovirus vectors of the invention can be made in accordance with standard recombinant DNA techniques. In general, the vectors are made by making a plasmid comprising a desired transcription unit inserted into a suitable adenovirus genome fragment. The plasmid is then co-transfected with a linearised viral genome derived from an adenovirus vector of interest and introduced into a recipient cell under conditions favouring homologous recombination between the genomic fragment and the adenovirus vector. Preferably, the transcription unit is engineered into the site of an adenovirus E1 deletion. Accordingly, the transcription unit is inserted into the adenoviral genome at a predetermined site, creating a recombinant adenoviral vector. The recombinant adenovirus vector is further recombined with additional vectors comprising desired E3 and/or E4 cassettes to produce the adenovirus vectors. The recombinant adenovirus vectors are encapsidated into adenovirus particles as evidenced by the formation of plaques in standard viral plaque assays. Preparation of replication-defective adenovirus stocks can be accomplished using cell lines that complement viral genes deleted from the vector, (*e.g*., 293 or A549 cells containing the deleted adenovirus E1 genomic sequences). After amplification of plaques in suitable complementing cell lines, the viruses can be recovered by freeze-thawing and subsequently purified using cesium chloride centrifugation. Alternatively, virus purification can be performed using chromatographic techniques. Examples of such techniques can be found for example in published PCT application WO/9630534, and Armentano *et al.,* 1993 (each reference incorporated herein by reference).

In a preferred embodiment, the mammal of the invention is an adult mouse and the amount of adenovirus particules administered to deplete or inhibits at least some antigen presenting cells of said adult mouse is equal or greater to 10¹⁰, 2 X 10¹⁰, 4 X 10¹⁰ 4.5 X 10¹⁰, 5 X 10¹⁰, 5.5 X 10¹⁰, 6 X 10¹⁰, 6.5 X 10¹⁰, 7 X 10¹⁰, 7.5 X 10¹⁰, 8 X 10¹⁰ , 8.5 X 10¹⁰ , 9 X 10¹⁰ , 9.5 X 10¹⁰ , 10¹¹ particles, said particles comprising optionally said additional adenovirus. In a preferred embodiment, the amount of adenovirus particles is greater than 6 X 10¹⁰ particles. The determination of the concentration of a particule in a viral stock can be performed by using absorbance at 260 nm or 280 nm optical density or alternatively by electron microscopy. Even if the amount of recombinant adenovirus able to form plaque doesn't seem to be determinant for the induction of tolerisation, it is important to trigger an immune response. That's the reason why it is highly important to evaluate the contamination of the viral stock prior to perform a method of the invention. Indeed, the inventors showed in the following examples that one can observed variations in the amount of recombinant virus expressed in pfu/mouse to trigger tolerization, these variations being caused by differences in the contamination of the viral stocks, some viral stocks having a greater concentration of non-competent viruses or wild type viruses than others. Nevertheless, the amount of recombinant adenovirus able to form plaque, should be preferably equal or greater to 2 X 10⁹ pfu/adult mouse, 2.5 X 10⁹ pfu/adult mouse, 3 X 10⁹ pfu/adult mouse, 3.5 X 10⁹ pfu/adult mouse, 4 X 10⁹ pfu/adult mouse, 4.5 X .10⁹ pfu/adult mouse, 5 X 10⁹ pfu/adult mouse, 6 X 10⁹ pfu/adult mouse, 8 X 10⁹ pfu/adult mouse, 10¹⁰ pfu/adult mouse. More preferably is greater than 4.10⁹ pfu/adult mouse. Titers of replication-defective adenoviral vector stocks expressed in pfu (plaque-forming unit) can be determined by plaque formation in a complementing cell line, *e.g*., 293 cells. For example, end-point dilution using an antibody to the adenoviral hexon protein may be used to quantitate virus production (Armentano *et al.,* 1995).

The invention also provides a method of producing transgenic mammal expressing an heterologous protein said method comprising the step of inhibiting in said mammal formation of neutralizing antibodies directed against said heterologous protein by the use of the previous method thereby allowing a long-lasting expression of said heterologous protein. In such method of producing transgenic mammal, said mammal is selected among domestic livestock, such as cow, pig, goat, sheep, horse, or laboratory animal, such as mouse, rat, rabbit, chinese pig, hamster, or pet animal such as cat and dog.

By "long-lasting expression of said heterologous protein" it is meant at least the time for the host immune system to produce neutralizing antibodies against said agent, usually 2 ou 3 weeks. More preferably, a long lasting expression as used herein means an expression with a duration greater than 3 weeks, 1 month, 2 months, 4 months, 6 months, 8 months, 10 months, or greater than one year. Assays suitable for use to determine persistence of transgene expression include measurement of transgene mRNA (*e.g*., by Northern blot, S1 analysis, reverse transcription-polymerase chain reaction (RT-PCR)), or incorporation of detectably-labelled nucleotide precursors (*e.g*., radioactively or fluorescently labelled nucleotide precursors) or by biological assays, such as a plaque assay, *e.g*., for a transgene encoding an essential viral gene product in a non-permissive cell line). Additionally, presence of a polypeptide or protein encoded by the transgene may be detected by Western blot, immunoprecipitation, immunocytochemistry, radioimmunoassay (RIA) or other techniques known to those skilled in the art. In general, transgene persistence can be evaluated *in vivo* or *in vitro* using several test formats. For example, cell lines can be transfected with plasmids, adenovirus vectors, or infected with recombinant adenoviruses of the invention. These assays generally measure the level and duration of expression of a contained transgene. Examples of such assays have been reported in Armentano *et al*.(1997). Additionally, persistence of transgene expression may also be measured using suitable animal models. Animal models are particularly relevant to assess transgene persistence against a background of potential host immune responses. Such a model may be chosen with reference to such parameters as ease of delivery, identity of transgene, relevant molecular assays, and assessment of clinical status. Where the transgene encodes a therapeutic protein, an animal model which is representative of a disease state may optimally be used in order to assess clinical improvement.

The invention is also dedicated to provide a method for reducing an anti-heterologous protein immune response in a mammal, including human, subject to the administration of said heterologous protein and/or nucleic acid sequence encoding said heterologous protein, said method comprising the step of inhibiting in said mammal formation of neutralizing antibodies directed against said heterologous protein by the method of the invention. Said method can be a step of a gene therapy protocol for the treatment of a mammal, preferably a human, afflicted with a disease selected among inheritated or acquired genetic diseases, infectious diseases such as viral infections, bacterial infections, parasital infections, funguses infections, and sceptic shocks, inflammatory diseases, autoimmune diseases, cancers, and their associated syndromes thereof.

More precisely, the invention provides a method for the therapy of a mammal, including humans, afflicted with a disease characterized by the altered expression of an endogenous protein, said method comprising the step of administering to said mammal said protein and/or nucleic acid sequence encoding said protein, and simultaneously or previously, the step of inhibiting in said mammal formation of neutralizing antibodies directed against said protein by the method of the invention. In an alternative way, the method of the invention further comprises the step of co-administering simultaneously, separately or sequentially, to said mammal at least one immune modulators such as cyclosporin, cyclophosphamide, desoxyspergualine, FK506, interleukin-4, interleukin-12, interferon-gamma, anti-CD4 monoclonal antibody, anti-CD8 monoclonal antibody, anti-LF1 antibody, antibody directed against CD40 ligand or CTLA4 Ig and the like.

The invention provides a method of modulating in a mammal formation of neutralizing antibodies directed against an heterologous protein, said method comprising the steps of :
(i) Optionally, co-administering to a first mammal, at least one agent and said heterologous protein and/or a nucleic acid sequence encoding said heterologous protein, said agent being administered simultaneously, sequentially or separately with said heterologous protein and/or nucleic acid sequence, and determining at least one amount of said heterologous protein and said agent, sufficient to trigger an immune response against said heterologous protein by said first mammal; optionally, re-performing step (i) until said amount is determined ;
(ii)co-administering to a second mammal said heterologous protein and/or a nucleic acid sequence encoding said heterologous protein, in an amount sufficient to trigger an immune response against said heterologous protein, as determined at step (i) and prior or simultaneously, said agent, in an amount greater that the one determined at step (i) and sufficient to trigger an immune response against said agent and sufficient to deplete or inhibit at least some antigen presenting cells of said mammal, and determining for said second mammal at least one amount of said agent that reduces and/or suppresses the anti-heterologous protein immune response in said mammal; re-performing step (ii) until said amount is determined ; and
wherein,
(a) when one administers to a third mammal, said agent in an amount equal or greater than the one determined at step (i) but lesser than the one determined at step (ii), said mammal produces neutralizing antibodies against said heterologous protein and optionally against said agent; and
(b) when one administers to said mammal said agent in an amount equal or greater than the one determined at step (ii), said mammal produces neutralizing antibodies against said agent but produces no or few neutralizing antibodies against said heterologous protein.

In a preferred embodiment, the amount of said agent of step (ii) is at least twice, 2.5 times, 3 times, 3.5 times, 4 times, 5 times, 6 times, 7 times, 8 times, 10 times the amount of said agent determined at step (i).

In a preferred embodiment of said method of the invention of modulating in a mammal formation of neutralizing antibodies directed against an heterologous protein, said mammal is a mouse and said agent is an adenovirus, and said agent and said nucleic acid sequence encoding said heterologous protein are simultaneously co-administered as a recombinant adenovirus, the genome of which comprising at least said nucleic acid sequence encoding said heterologous protein. Moreover, the amount of said recombinant adenovirus particles of step (i) that triggers an immune response towards said heterologous protein in said mouse without depleting or inhibiting at least some antigen presenting cell of said mouse is below 4.10¹⁰ particles, and/or the amount of said adenovirus particles able to form plaque is below 4.10⁹ pfu/mouse; and the amount of said recombinant adenovirus particles of step (ii) that reduces or suppresses the anti-heterologous protein immune response in said mouse is at least equal or greater than 4.10¹⁰ particles and/or the amount of said adenovirus particles able to form plaque is equal or greater than 4.10⁹ pfu/mouse.

This method of the invention of modulating in a mammal formation of neutralizing antibodies directed against an heterologous protein can further comprise the step of administering an additional agent to said mammal in step (i) and (ii).

It is also the goal of the present invention to use the method of the invention to inhibit in a mammal formation of neutralizing antibodies directed against an heterologous protein, said method comprising the step of co-administering to said mammal, said heterologous protein and/or a nucleic acid sequence encoding said heterologous protein and prior or simultaneously said agent in an amount equal or greater than the one determined at step (ii).

It is also the goal of the present invention to use the method of the invention to trigger in a mammal formation of neutralizing antibodies directed against an heterologous protein, said method comprising the step of co-administering simultaneously, separately or sequentially to said mammal said heterologous protein and/or a nucleic acid sequence encoding said heterologous protein, and said agent in an amount and/or concentration equal or greater than the one determined at step (i) but lesser than the one determined at step (ii).

The invention also provides a method for the therapy of a mammal affected by a disease wherein at least one endogenous protein is involved in said disease ethiology, said method comprising the step of inhibiting the biological functions of said endogenous protein by enhancing the production of neutralizing antibodies against said protein by use the method of the invention. For example, said disease can be selected among inherited or acquired genetic diseases, auto-immune diseases, cancers, inflammatory diseases, infectious diseases such as viral infections, bacterial infections, parasital infections, funguses infections, sceptic shocks and their associated syndromes and complications thereof.

Among inherited genetic diseases, one can recite Duchenne muscular dystrophy, Steinert syndrome, retinoblastoma, glaucoma, spino muscular atrophy.

Among auto-immune diseases of the invention, one has to recite psoriasis, atopic dermatitis, contact dermatitis, cutaneous T cell lymphoma (CTCL), Sezary syndrome, pemphigus vulgaris, bussous pemphigoid, erythema nodosum, scleroderma, uveitis, Bechet's disease, sarcoidosis Boeck, Sjögren's syndrome, rheumatoid arthritis, juvenile arthritis, Reiter's syndrome, gout, osteoarthrosis, systemic lupus erythematosis, polymyositis, myocarditis, primary biliary cirrhosis, Crohn's disease, ulcerative colitis, multiple sclerosis and other demyelinating diseases, aplastic anaemia, idiopathic thrombocytopenic purpura, multiple myeloma and B cell lymphoma, Simmon's panhypopituitarism, Graves' disease and Graves' opthalmopathy, subacute thyreoditis and Hashimoto's disease, Addison's disease, insulin-dependent diabetes mellitus (type 1).

Among cancers, one can recite solid tumors such as head and neck cancers, lung cancer, gastrointestinal track cancer, breast cancer, gynecologic cancer, testicular cancer, urinary tract cancer, neurologic tumors, endocrine neoplasms, skin cancers (melanoma...), sarcomas, and also hematologic malignacies such as Hodgkin's disease and malignant lymphomas, immunoproliferative diseases, chronic leukemias, myeloproliferative disorders, acute leukemias and also pre-tumoral syndromes.

The invention also provides a method for the therapy of a mammal affected by a chronic or an acute infection, such as a sceptic shock.

Examples of viral infections are the ones induced by the human immunodeficiency virus (HIV), the hepatitis B virus, the hepatitis C virus, the parainfluenza virus, the herpes virus type 1 and 2 (HSV 1, HSV 2), the cytomegalovirus, the Epstein Barr virus (EBV), the varicella zona virus, the papillomavirus, the human T leukemia virus 1 and 2 (HTLV1 and HTLV2), the myxovirus, the poliovirus, the coxsackie virus A and B, the echovirus, the enterovirus, the rhinovirus, the rhabdivirus, the arbovirus, the hemorragic fever viruses, the poxvirus infections.

Examples of bacterial infections are the ones induced by *Helicobacter pylori, Escherichia coli,* Klebsiella, Enterobacter, Serratia, Proteus, Pseudomonas æruginosa, Acinetobacter, Bacteroides, Fusobacterium, Leptotrichia, Propionibacterium, Eubacterium, Actinomyces, Veillonella, Clostridium, Leptospira, Borrelia, Treponema, Mycobacterium tuberculosis, Mycobacterium bovis, atypical Mycobacterium, Rickettsia, Coxiella, Mycoplasma pneumoniae, staphylococcus, steptococcus, pneumococcus, Neisseria meningitidis, Corynebacterium diphteriae, Listeria monocytogenes, Hæmophilus influenzæ, Brucella melitensis, Brucella *abortus bovis,* Brucella *abortus suis,* Yersinia pseudotuberculosis, Yersinia enterocolitica, Yersinia *pestis,* Salmonella typhi, Salmonella paratyphi, Salmonella typhi murium.

Examples of parasites infections are the ones induced by *schistosoma mansoni,* Schistosoma *intercalatum,* Schistosoma hæmatobium, Schitosoma *japonicum,* Schistosoma mekongi, distomatosis, Toxoplasma gondii, Rickettsia, Pneumocystis carinii, Piroplasmosis, Echinococcus, Wuchereria bancrofti, Brugia malayi.

Examples of funguses infections are the ones induced by *Candida albicans,* Candida *tropicalis,* Candida pseudotropicalis, Candida krusei infections, Candida parapsilosis, Candida guillermondii, Aspergillosis, Cryptococcus neoformans.

Among inflammatory diseases, one has to recite inflammatory arthritis, Crohn's disease, rectocolitis.

The invention also provides the use of a method of the invention to produce a mammal with a functional inactivation of at least one endogenous protein, said method comprising the step of administering to a mammal in a simultaneous, separate or sequential manner at least one agent and an heterologous protein and/or a nucleic acid sequence encoding for said heterologous protein, said nucleic acid sequence being expressed in at least one cell of said mammal, wherein said heterologous protein being substantially identical to said endogenous protein wherein the amount of said heterologous protein, optionally of said agent, that is administered to said mammal is the one determined in step (i), thereby the amount of anti-heterologous neutralizing antibodies produced by said mammal being sufficient to alter the biological activity of said heterologous protein and /or of said endogenous protein.

In a preferred embodiment, the mammal of the invention is an adult mouse and the amount of recombinant adenovirus particles administered to produce neutralizing antibodies against said heterologous proteins is equal or below 2.10¹⁰ particles, 10¹⁰, 9.10⁹, 8.10⁹, 7.10⁹, 5.10⁹, 3.10⁹, 2.10⁹, 10⁹, 5.10⁸ particles.

To produce a mammal with a phenotype of a functional inactivation by the method of the invention, said heterologous protein is at least 10%, 15%, 20%, 30%, 40%, 50%, 55%, 60%, 65%, 70%, 75%, 80%, 85%, 87.5%, 90%, 92.5%, 95%, 96%, 97%, 98%, 99%, 99.5%, 99.9% identical to the endogenous protein. In a preferred embodiment, said heterologous protein is at least 50% identical to the endogenous protein. Said heterologous protein is preferably a protein selected among animal species, such as rabbit, mouse, rat, preferably humans, homologous or substantially identical to said endogenous protein of said mammal, more preferably of said mouse. Example of human protein is hGH (human growth hormone) that is substantially identical to the murine growth hormone.

As used herein, "percentage of identity" between two nucleic acids sequences or two amino acids sequences, means the percentage of identical nucleotides, respectively amino-acids, between the two sequences to be compared, obtained with the best alignment of said sequences, this percentage being purely statistical and the differences between these two sequences being randomly spread over the nucleic acids or amino acids sequences. As used herein, " best alignment" or "optimal alignment", means the alignment for which the determined percentage of identity (see below) is the highest. Sequences comparison between two nucleic acids or amino acids sequences are usually realised by comparing these sequences that have been previously align according to the best alignment; this comparison is realised on segments of comparison in order to identify and compared the local regions of similarity. The best sequences alignment to perform comparison can be realised, beside by a manual way, by using the local homology algorithm developped by Smith and Waterman (1981), by using the local homology algorithm developped by Neddleman and Wunsch (1970), by using the method of similarities developped by Pearson and Lipman (1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA). To get the best alignement, one can preferably used BLAST software, with the BLOSUM 62 matrix, or the PAM or PAM 250 matrix. The identity percentage between two sequences of nucleic acids or amino acids is determined by comparing these two sequences optimally aligned, the nucleic acids or the amino acids sequences being able to comprise additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

As used herein amino acids sequences, and respectively nucleic acids sequences, having a percentage of identity of at least 10%, preferably, at least 15%, 20%, 30%, 40%, 50%, 55%, 60%, 65%, 70%, 75%, 80%, 85%, 87.5%, 90%, 92.5%, 95%, 96%, 97%, 98%, 99%, 99.5%, 99.9% after optimal alignment, means amino acids sequences, respectively nucleic acids sequences, having with regard to the reference sequence, modifications such as deletions, truncations, insertions, chimeric fusions, and/or substitutions, specially point mutations, the amino acids sequence, respectively nucleic sequence, of which presenting at least 10%, 15%, 20%, 30%, 40%, 50%, 55%, 60%, 65%, 70%, 75%, 80%, 85%, 87.5%, 90%, 92.5%, 95%, 96%, 97%, 98%, 99%, 99.5%, 99.9% identity after optimal alignment with the amino acids sequence, respectively, nucleic acid sequence of reference.

In another preferred embodiment, the invention provides a method of producing a functional inactivation of an endogenous protein in animal by inactivating at least one endogenous protein, said method comprising the step of triggering in said mammal formation of neutralizing antibodies directed against an heterologous protein being substantially identical to said endogenous protein, said method comprising the step of co-administering to said mammal in a simultaneous, separate or sequential manner, at least one agent and said heterologous protein and/or a nucleic acid sequence encoding for said heterologous protein, said nucleic acid sequence being expressed in at least one cell of said mammal, wherein the amount of said heterologous protein, optionally of said agent, is at least sufficient to trigger an immune response against said heterologous protein and the amount of said agent is not sufficient to deplete or inhibite at least some antigen presenting cells of said mammal.

The nucleic acid sequence of the invention undifferently named transgene, gene, vector in the present invention, can be used to transiently transfect or transform host cells, or can be integrated into the host cell chromosome. Preferably, however, the nucleic acid sequence can include sequences that allow its replication and stable or semi-stable maintenance in the host cell. Many such sequences for use in various eukaryotic cells are known and their use in vectors routine. Generally, it is preferred that replication sequences known to function in host cells of interest be used.

Preferably the nucleic acid sequence of the invention contains all the genetic information needed to direct the expression of said heterologous protein in at least one cell of the mammal, preferably in at least one APC cell of the mammal such as promoter sequences, regulatory upstream elements, transcriptional and/or translational initiation, termination and/or regulation elements. Various promoters, including ubiquitous or tissue-specific promoters, and inducible and constitutive promoters may be used to drive the expression of the heterologous protein gene of the invention. Preferred promoters for use in mammalian host cells include strong viral promoters from polymoma virus, Simian Virus 40 (SV40), adenovirus, retroviruses, *hepatitis* B virus, *herpes simplex* virus (HSV), Rous *sarcoma* virus (RSV), mouse mammary tumor virus (MMTV), and most preferably cytomegalovirus (CMV), but also heterologous mammalian promoters such as the - actin promoter, phosphoglycerate kinase (PGK) promoter, epithelial growth factor 1 (EGF1 ) promoter, albumin promoter, creatine kinase promoter, methall-thionein promoter. In preferred embodiments, the promoters are chosen among cytomegalovirus early promoter (CMV IEP), Rous sarcoma virus long terminal repeat promoter (RSV LTR), myeloproliferative sarcoma virus long terminal repeat (MPSV LTR), simian virus 40 early promoter (SV40 IEP), and major late promoter of the adeovirus. Alternatively, other eukaryotic promoters are suitable for such use, including elongation factor one-alpha (EF1- ) promoter, creatinine kinase promoter, albumine promoter, phosphoglycerate kinase promoter. Inducible promoters such as tertacycline promoters could also be used. Transcription of the gene encoding the heterologous protein can be increased by inserting an enhancer sequence into the vector. Many enhancer sequences are now known from mammalian genes (globin, elastase, albumin, and insulin) or from eukaryotic cell virus (SV40, CMV). The disclosed vectors preferably also contain a polyadenylation signal. All of the above mentioned regulation sequences are operably linked to provide optimal expression of the transgene.

The heterologous protein of the invention or a fragment thereof is selected among the proteins presented by a class I major histocompatibility molecule (CMH I), a class II major histocompatibility molecule (CMH II), or by both class I major histocompatibility molecule and class II major histocompatibility molecule.

The heterologous protein of the present invention can be any non-endogenous protein. The heterologous protein can be selected among protein from different species homologous to the endogenous protein, mutated and/or truncated endogenous protein, protein exhibiting a polymorphism compared to the endogenous protein, fusion protein with said endogenous protein. More preferably, said heterologous protein is chosen among secreted proteins, membranes proteins, receptors, intracellular proteins, nuclear proteins. Examples of secreted heterologous proteins are neuromediators, hormones such as parathyroïd hormone-related protein, cytokines such as interleukines such as interleukin 1 (Il-1), interleukin 6 (Il-6), interleukin 21 (Il-21), lymphokines, interferons, chemokines such as tumor necrosis factor (TNF), monokines, growth factors, blood derivatives, neurotransmitters. Examples of proteins of a particular therapeutical interest are CFTR, dystrophin, growth hormone, insulin, insulin growth factor 1 and 2, tumor necrosis factor, blood factor VIII, blood factor IX, ACTH receptor.

The heterologous protein of the invention can also be a reporter protein. Among reporter proteins one can recite -galactosidase, luciferase, autofluorescence protein, such as the green fluorescence protein (GFP).

In one embodiment, the heterologous protein of the invention is mutated in order to enhance its immunogenicity. Such mutation(s) in the nucleic acid sequence encoding said heterologous proteins are selected in a group consisting of naturally occurring mutation, genetically engineered mutation, chemically induced mutation, physically induced mutation. In a preferred embodiment mutation is induced by recombinant DNA techniques known in the art. For example, it may include among others, site directed mutagenesis or random mutagenesis of DNA sequence which encodes said protein. Such methods may, among others, include polymerase chain reaction (PCR) with oligonucleotide primers bearing one or more mutations (Ho *et al.,* 1989) or total synthesis of mutated genes (Hostomsky *et al.,* 1989). These methods can be used to create variants which include, *e.g*., deletions, insertions or substitutions of residues of the known amino acids sequence of the heterologous protein of the invention. PCR mutagenesis using reduced Taq polymerase fidelity can also be used to introduce random mutations into a cloned fragment of DNA (Leung *et al.,* 1989). Random mutagenesis can also be performed according to the method of Mayers *et al.,* 1985). This technique includes generations of mutations, *e.g*., by chemical treatment or irradiation of single-strand DNA *in vitro,* and synthesis of a complementary DNA strand. Alternatively fragment of an immunogenic peptide from bacteria, virus for example can be inserted throughout the protein.

The host animal, preferably the mammal, obtained by the method of the invention of producing functional inactivation of an endogenous protein is also in the scope of the invention. Such mammal is preferably chosen among domestic lifestock, pet animals as previously described or among laboratory animals like for example, mouse, rat, rabbit, Chinese pig, hamster, dwarf pig, monkeys and others. More preferably, the animal is a mouse; suitable mouse strains are available that are either inbred (*i.e*. 129Sv, C57B16, Balb/c,...) or oubred. Such mice could react differently to the co-administration of an agent and a heterologous protein and/or a nucleic acid sequence encoding said heterologous protein according to their genetic background. It could be useful to optimize the amount of atent and heterelogous protein of the invention to modulate theh production of neutralizing antibodies for each mouse background. For example, C57/b16 mice do not trigger an efficient immune response against the adenoviral particle but DBA/2J does trigger an efficient response. Such animals with a functional inactivation phenotype, especially such mice, are very useful to perform biological, physiological, biochemical, molecular studies and analysis of the function of said heterologous and/or homologous protein.

It is also a goal of the invention to use the mammal obtained by the above described method to perform drug screening.

The use of a mammal obtained by the above described method to isolate spleen cells from said mammal that expresses antibody directed against said heterologous an/or endogenous protein to make hybridoma(s)is also in the scope of the invention. Alternatively, the biological fluid of the mammal of the invention can be used to prepare serum and/or polyclonal antibodies.

The therapeutical composition comprising at least the agent and the heterologous protein and/or nucleic acid sequence of the invention with « pharmaceutically acceptable carriers » is also in the scope of the invention. Such composition is adapted according to the therapeutical needs of the animal, preferably of the human patient. For example, to treat a disease wherein the biological activity of a endogenous protein (i.e a tumoral marker, an over-expressed protein, ...) has to be inhibited or shut off, a composition of the invention can be used to generate neutralizing antibodies against said endogenous protein. Alternatively, the composition of the invention is highly desirable to allow a long-lasting expression of a protein in a patient in need of such a treatment (i.e to correct an inheritated disease, to regulate hormonal secretion, to stimulate the immune system, etc...). Preferably, the heterologous protein of the invention is a secreted protein.

It is also in the scope of the invention to provide a method to produce vaccine for a mammal, against an heterologous protein, said method comprising the step of triggering in said mammal formation of neutralizing antibodies directed against said heterologous protein, by using the method of the invention. These vaccines may either be prophylactic (to prevent infection) or therapeutic (to treat disease after infection). Such vaccines comprises the agent and the heterologous protein of the invention and/or nucleic acid encoding said heterologous protein, more preferably the recombinant adenovirus of the invention, usually in combination with « pharmaceutically acceptable carriers », which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipids aggregates (such as oil droplets or liposomes), and inactive virus particle. Such carriers are well known to those of ordinary skill in the art. Additionally, such carriers may function as immunostimulating agents also called « adjuvants » ; preferred adjuvants to enhance effectiveness of the composition include, but are not limited to : (1) aluminium salts (alum), such as aluminium hydroxyde, aluminium phosphate, aluminium sulfate, etc. ; (2) oil-in-water emulsion formulations, such as for example MF59 (WO 90/14 837), SAF, Ribi^{™} adjuvant system (Ribi Immunochem, Hamilton, MT USA) ; (3) saponin adjuvants; (4) complete Freunds adjuvant (CFA) and incomplete Freunds adjuvant (IFA) ; (5) cytokines such as interleukines (Il-1, Il-2, etc.), macrophage colony stimulating factor (M-CSF), tumor necrosis factor (TNF) etc. ; (6) other substances that act as stimulating agents to enhance the effectiveness of the composition. The vaccines are conventionally administered parenterally, *e.g*., by injection, either subcutaneously or intramuscularly. Additional formulations suitable for other modes of administration include oral and pulmonary formulations, suppositories, and transdermal applications. Dosage treatment may be single dose schedule or a multiple dose schedule. The vaccine may be administered in conjunction with other immunoregulatory agents.

Dosage of the agent and of the heterologous protein and/or nucleic acid sequence of the invention to be administered to an animal or an individual for persistent expression of a transgene encoding at least a biologically active protein for animal transgenesis or human gene therapy and to achieve a specific inactivation phenotype is determined with reference to various parameters, including the animal species, the condition to be treated, the age, weight and clinical status of the individual, and the particular molecular defect requiring the provision of a biologically active protein. In a preferred embodiment, the agent and the nucleic acid sequence encoding the heterologous protein corresponds to a recombinant virus, the genome of which encoding said heterologous protein and the mammal is a mouse. A man skilled in the art will know by using the method of the invention how to determine the amount of agent and the amount of nucleic acid sequence encoding said heterologous protein, preferably said recombinant adenovirus, required either to induce a long-lasting expression of the heterologous protein, or to functionally inactivate an endogenous protein, in human, or in another mammal.

The dosage is preferably chosen so that administration causes a specific phenotypic result, as measured by molecular assays or clinical markers. For example, determination of the persistence of the expression of a transgene encoding said heterologous protein which is administered to an animal or an individual as a recombinant adenovirus can be performed by molecular assays including the measurement of heterologous protein mRNA, by, for example, Northern blot, S1 or RT-PCR analysis or the measurement of the heterologous protein as detected by Western blot, immunoprecipitation, immunocytochemistry, or other techniques known to those skilled in the art. For example, determination of the functional inactivation of an endogenous protein can be performed by a phenotypic analysis, by an altered biological activity of the endogenous protein.

The administration of said agent and said heterologous protein and/or nucleic acid sequence encoding said heterologous protein is performed via a technique chosen among intravenous injection, intravaginal injection, intrarectal injection, intramuscular injection, intradermic injection. Preferably, the administration is performed via intravenous injection, selected among retro-orbital sinus injection, tail injection, hepatic injection, femoral or jugular injection. Hepatic injection is the most preferred because of the homogenous distribution and the accessibility of APC's in the liver. Single injection or multiple injections at the same or at different loci can be performed in order to increase transgene expression and/or enhance the depletion and/or inactivation of the APC's cells.

Maximum benefit and achievement of a specific phenotypic result from administration of the agent and the heterologous protein and/or nucleic acid sequence encoding said heterologous protein of the invention may require repeated administration. Where a viral vector -especially an adenoviral- is used to deliver some or all of the components of the transgene expression vector, such repeated administration may involve the use of the same adenoviral vector, or, alternatively, may involve the use of different vectors which are rotated in order to alter viral antigen expression and decrease host immune response.

The practice of the invention employs, unless other otherwise indicated, conventional techniques or protein chemistry, molecular virology, microbiology, recombinant DNA technology, and pharmacology, which are within the skill of the art. Such techniques are explained fully in the literature. (See Ausubel et al., 1995, Current Protocols in Molecular Biology, Eds., John Wiley & Sons, Inc. New York, Remington's Pharmaceutical Sciences, 17th ed., Mack Publishing Co., Easton, Pa., 1985, and Sambrook *et al.,* 1989).

Unless defined otherwise, all technical and scientific terms used herein have the same meaning as is commonly understood by one of the skill in the art to which this invention belongs.

The figures and examples presented below are provided as further guide to the practitioner of ordinary skill in the art and are not to be construed as limiting the invention in anyway.

### EXAMPLES

### 1. MATERIALS AND METHODS

### 1.1. Construction of recombinant E1-deleted adenovirus vector

The huTPO cDNA was inserted in the EcoRV restriction site of the adenovirus (Ad) Rous sarcoma virus (RSV) β-galactosidase (ßgal) plasmid after excision of the ßgal gene by SalI. The huTPO cDNA under control of the RSV viral promoter is followed by a fragment of Ad5 (mu 9.4-17; BglII-HindIII) to permit homologous recombination for the generation of the recombinant adenovirus AdRSVhuTPO. The resulting plasmid was cotransfected into the human embryonic 293 cell line with ClaI-digested Ad5d1324 DNA using precipitation by calcium phosphate, as previously described (Stradford-Perricaudet *et al.,* 1990). AdRSVßgal carrying the nuclear localization site *Escherichia coli* lacZ marker gene under the control of the same viral promoter was used as a control and has been previously described *(Stradford-Perricaudet et al.*,1990). Viral stocks were prepared by infection of the 293 cell line, purified and concentrated by a double cesium chloride gradient, dialyzed, aliquoted, and stored in 10% glycerol at -80°C. Titers of the viral stocks were determined by limiting dilution on plaque assays using 293 cells and expressed as PFU. The total number of viral particles was quantified by optical density at 260 nm of an aliquot of the virus stock diluted in virion lysis solution (0.1% SDS, 10 mM Tris-HCl, 1 mM EDTA).

### 1.2. Animal procedures

DBA/2J-specific pathogen-free mice were obtained from Janvier (Orleans, France). All animals were bred in negative pressure isolators for adenovirus injection experiments in the animal facilities of Institut Gustave Roussy (Villejuif, France). Female mice (6-8 wk old) were injected with recombinant adenoviruses via the retroorbital sinus. DBA/2J mice were injected with 3 to 6 x 10⁹ PFU of AdRSVhuTPO, while control mice were injected with the same doses of AdRSVßgal or with PBS.

### 1.3. TPO concentrations

Serum TPO concentrations were measured using a microwell assay (Gough *et al.,* 1985). Assays were performed in duplicate by adding 200 cells from the human c-mpl-transfected Ba/F3 cell line (Wendling *et al.,* 1994) in a 10-µl vol of DMEM plus 10% FCS to serial twofold dilutions of the serum. TPO concentrations were calculated by assigning 1 U/ml to the concentration, resulting in 50% cell survival after 2 to 3 days of incubation at 37°C in a humidified atmosphere of 10% CO2 in air. In a dose-response analysis using the full-length rhuTPO, 1 U is approximately the equivalent of 100 pg of the molecule.

### 1.4. Peripheral blood hematologic measurements

Blood samples were obtained from ether-anesthetized animals by puncture of the retroorbital sinus. After RBC lysis in Unopette vials (Becton Dickinson, Franklin Lakes, NJ), platelets and white cells were counted by microscopy and microhematocrits were determined following blood centrifugation.

### 1.5. Analysis of clonogenic committed progenitor cells

Femoral marrow (8 x 10⁴) and spleen cells (1 x 10⁶) of DBA/2J mice, harvested at various times following injection of AdRSVhuTPO, were cultured in 1 ml of 0.8% methylcellulose in Iscove's medium supplemented with 20% FCS supplemented with rmuIL-3 (100 U/ml; Immunex, Seattle, WA) and rhuEpo (1 U/ml; Cilag, Paris, France) to determine the number of granulocyte-macrophage CFU (CFU-GM) and erythroid burst-forming cells (BFU-E). Megakaryocyte CFU (CFU-MK) were grown in 0.3% agar supplemented with rmuTPO (10 ng/ml; ZymoGenetics, Seattle, WA), rmuIL-3, and recombinant murine stem cell factor (50 ng/ml; Immunex), as previously described, using 1 x 10⁵ marrow cells and 5 x 10⁵ spleen cells/500 µl agar medium (Wendling *et al.,* 1994). For each determination, cultures for one non-injected and one AdRSVhuTPO-injected mouse were performed in duplicate at 37°C/5% CO₂ in air for 5 days.

### 1.6. TPO-neutralizing activity in the sera of thrombocytopenic mice

To determine the anti-TPO activity in the sera of thrombocytopenic mice, microwell assays were performed by adding 200 cells from the human or murine c-mpl-transfected Ba/F3 cell line to serial dilutions of the serum previously incubated for 1 h at 37°C with 2 U/ml (200 pg/ml) of rhuTPO or rmuTPO, respectively. rhuTPO was added at a high concentration (5 µg/ml) to serial dilutions of the serum to reverse the neutralization. To exclude nonspecific toxicity of the mouse serum, Ba/F3-mpl-transfected cells were also stimulated with 50 U/ml of rmuIL-3 added to the serial dilutions of the sera to be tested. All dilutions were tested in duplicate.

### 1.7. Detection of anti-human and anti-murine TPO Abs

Ninety-six-well Nunc Maxisorb plates were coated with 1 µg/ml of huTPO (Genzyme, Cambridge, MA) or muTPO (ZymoGenetics, Seattle, WA) in PBS/0.1% BSA overnight at 4°C. PBS/2% FCS was used to block nonspecific binding. Plates were washed (PBS/0.1% Tween-20), and serial dilutions of sera from AdRSVhuTPO- and AdRSVßgal-injected mice were incubated in the coated wells for 90 min at 37°C. The plates were washed five times with PBS/0.1% Tween-20 and then incubated with 100 µl of a 1/5000 dilution of peroxidase-conjugated goat anti-mouse IgG + IgM or goat anti-mouse IgM (Jackson ImmunoResearch Laboratories, West Grove, PA) for 1 h at 37°C. For determination of anti-huTPO Ab isotypes, the following peroxidase-conjugated Abs were used: goat anti-mouse IgG2a, goat anti-mouse IgG2b, and goat anti-mouse IgG1 (Southern Biotechnology, Birmingham, AL). All Abs were used at a dilution of 1/5000. Following washing, the wells were incubated with 100 µl of substrate (o-phenylenediamine-dihydrochloride; Sigma, St. Louis, MO). The reaction was stopped after 5 to 10 min by adding 50 µl of 12% H2SO4. The OD was measured with a spectrophotometer at 492 nm. Wells were considered as positive when the OD was approximatively twofold that of the OD observed with 5-wk serum from an AdRSVßgal-injected mouse. The IgG2a/IgG2b ratio was calculated by dividing the inverse of the last positive dilution of IgG2a anti-huTPO Ab by the inverse of the last positive dilution of IgG2b anti-huTPO Ab. For each mouse, the first dilution assayed was 1/40; if no positivity was found at this dilution, the titer was arbitrarily considered to be 1/10 for purposes of calculation.

### 1.8. Detection of anti-viral Abs

Microtiter plates as described above were coated for 18 h at 4°C with 100 µl/well of PBS containing 1 µg/ml of heat-inactivated AdRSVßgal particles treated with SDS (0.01%). Plates were washed (PBS/0.1% Tween-20), and serial dilutions of sera from AdRSVhuTPO- and PBS-injected mice were incubated in the coated wells for 90 min at 37°C. The plates were washed five times with PBS/0.1% Tween-20 and then incubated with 100 µl of a 1/5000 dilution of peroxidase-conjugated goat anti-mouse IgG + IgM for 1 h at 37°C. For determination of anti-adenoviral Ab isotypes, the same Abs used for determination of anti-TPO isotypes were used at the same dilutions.

### 1.9. Histology

Organs (spleen, femur, tibia, kidney, liver, and lung) of mice sacrificed at different times after the injection of the recombinant adenovirus vectors were fixed in Bouin's solution or buffered formaldehyde and embedded in paraffin. Thin sections (3-5 µm) were stained by hematoxylin/eosin (HE), May-Crünwald-Giemsa, or periodic acid-Schiff (PAS) stains. Long term -galactosidase expression in the liver was analyzed in mice injected with 8 x 10⁹ pfu of AdRSV gal by immuno-histochemistry in paraffin-embedded sections using a rabbit IgG fraction to - galactosidase (ICN Pharmaceuticals, Aurora, Ohio) at a 1/100 to 1/200 dilution.

### 2. RESULTS

### 2.1. Influence of the viral dose in the induction of a Long-term transgene expression or a functional inactivation of a homologous endogenous protein:

Mice were intraveinously injected with a TD (n=7) or an ID (n=8) of AdRSVhuTPO in two sets of separate experiments. Mice were weekly followed by the measure of blood platelets during 9 weeks. All mice injected with the ID of AdRSVhuTPO had initial increases in platelet counts within the first two weeks (median of 225±52 and 428±66 at week 1 and 2 respectively) followed by a reduction to low platelet levels starting as early as week 3 (median of 105±49, 93±54, 57±48, 59±35, 37.5±47, 41±57, 26±58 at week 3,4,5,6,7,8,9 respectively).

On the other hand mice injected with a TD of AdRSVhuTPO had as for the ID mice an increases in platelet counts during the first two weeks (median of 200±34 and 280±141 at week 1 and 2 respectively) but maintain this levels for the following weeks (median of 210±62, 175±121, 216±140, 241.5±130, 301.5±212, 218±100, 260±82 at week 3,4,5,6,7,8,9 respectively). A same viral preparation was used for the experiment with an ID at 2 x 10⁹ pfu and a TD at 6 x10⁹ pfu. Another viral preparation was used for the experiment with an ID at 4 x 10⁹ pfu and a TD at 8 x10⁹ pfu.

No platelet variation was observed during the follow-up in the PBS- or the AdRSV gal-injected mice. The mean platelet count was 144.2±12.3 x 10⁴/ 1 in the PBS-injected mice.

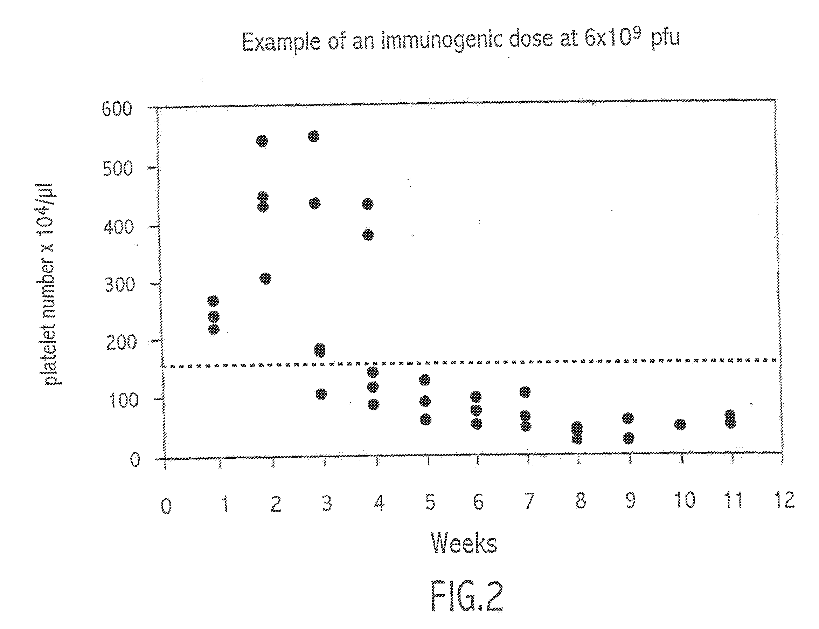

To further underline the role of the titer determination in the induction of the desire phenotype we included an experiment with an ID at 6 x 10⁹ pfu in the results ( Figure 2). Conversly to what previously described in mice injected by the same route with 6 x 10⁹ pfu mice showed a phenotype comparable to what observed with an ID of AdRSVhuTPO. In this experiment the virus stock was obtained from a diffrent preparation to the one giving a TD at the same pfu concentration. This result emphasize the importance for determining for each viral preparation the TD and the ID by a different way than the plate forming unit assays. Optical density at 260 nm with or without SDS lysis is suitable for this determination.

### 2.2. Human and murine TPO levels in the sera of mice injected with the ID of AdRSVhuTPO:

High levels of human TPO was detected by the bioactivity test on the human c-mpl-transfected Ba/F3 cell line during the first week, followed by a decline during the second and the third week after injection, and returned to undetectable levels after 4 week.

Since the murine TPO levels is physiologicaly inversly proportional to platelet levels, we measured the bioactivity of mice sera on the murine c-mpl-transfected Ba/F3 cell line when they became thrombocytopenic. No murine TPO bioactivity was measurable during the thrombocytopenic period.

### 2.3. TPO-neutralizing activity in the sera of thrombocytopenic mice:

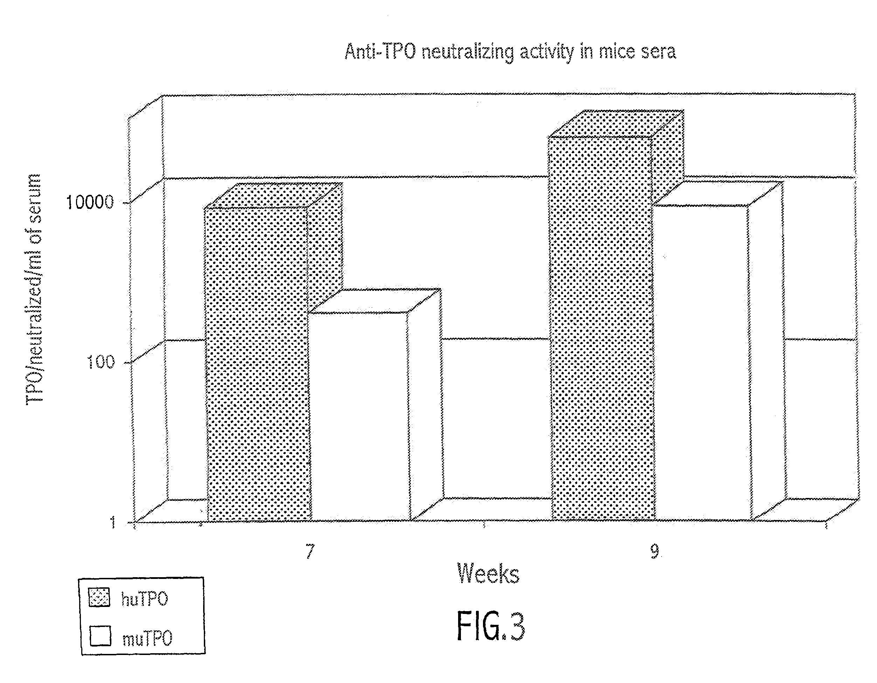

As shown in figure 3 , in a proliferation assay on a human or murine c-mpl-transfected Ba/F3 cell line a serum from a thrombocytopenic mouse is able to neutralize up to 32,000 Unit of human TPO and 8000 unit of murine TPO. This activity is enhanced with time.

### 2.4. Influence of the viral dose (ID or TD) in the induction of a humoral response against the human and murine TPO:

As a differiential kinetic expression of platelet was obtained with ID or TD of AdRSVhuTPO we analyzed anti-TPO antibodies in both groups at different times.

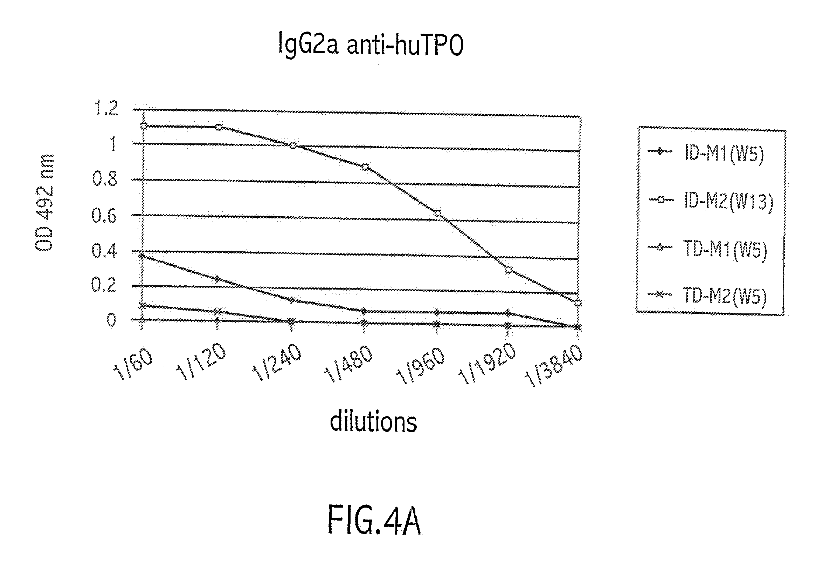

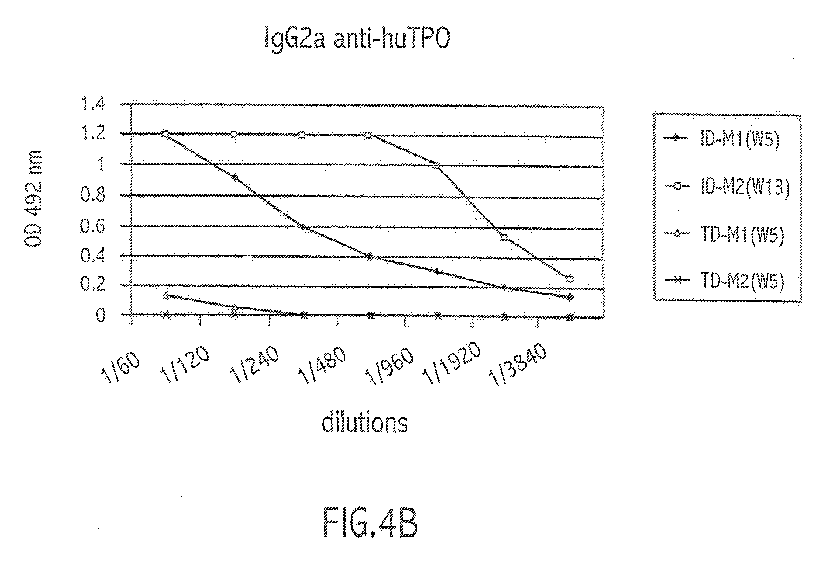

All thrombocytopenic mice obtained following injection of an ID of AdRSVhuTPO had a polyclonal anti-TPO antibody response (IgG1, IgG2a, IgM), while mice injected with a TD of AdRSVhuTPO had no anti-TPO antibody response (see figure 4A, 4B). Anti-TPO antibodies were cross-reactive, since hybridoma derived from thrombocytopenic recognized both human and murine TPO (see figure 5A, 5B, 5C).

### 2.5. Presence of a humoral response against the adenovirus capsid in mice injected with ID or TD of AdRSVhuTPO:

A similar polyclonal anti-adenovirus humoral response (IgG1, IgG2a) was observed following injection of mice with an ID of or a TD of AdRSVhuTPO (see figure 6A, 6B).

### 2.6. Efficient blocade by anti-TPO antibodies of all the physiologic functions of the endogenous TPO (murine):

Since thrombopoietin plays an important role in myeloid and erythroid progenitors beside of its major role all during the megacaryocitic lineage differentiation (Carver-Moore *et al.,* Alexander *et al*.) we analyzed the myeloid and erythroid clonogenic progenitors in thrombocytopenic mice at different time. As shown in table 1 all thrombopcytopenic mice had a reduction in myeloid and erythroid clonogenic progenitors both in the bone marrow (median values were 57,4%±12 and 35.7±14% of the value observed for the CFU-GM and BFU-E clonogenic progenitors in control mice respectively) and the spleen (39.6±14% and 33.3%±41 of the value observed for the CFU-GM and BFU-E clonogenic progenitors in control mice respectively). CFU-MK progenitors was also assayed at week 12 and showed 51 % and 19% of control values in the bone marrow and spleen respectively.

In addition histologic analysis of bone marrow and spleen of thrombocytopenic mice showed a significant decrease in megacaryocytic number in both tissues. In the marrow, megacaryocytes were estimated to be 10% of the values observed in control mice.

### 2.7. Long-term -galactosidase expression:

AdRSV gal was injected at an equivalent tolerigenic dose used for the AdhuTPO experiments, *i.e.* 8 x 10⁹ pfu. Immuno-histochemistry revealed - galactosidase expression in some hepatocytes in two mice and in the biliary duct in another mouse at 5 months. To date all the studies using an adenovirus vector encoding the -galactosidase (Yang *et al.,* 1994, 1994a, 1996) showed a complete elimination of transduced hepatocytes after 2 to 3 weeks.

### 3. COMMENTS

The present invention focuses on the use of a vector able to easily enter in antigen presenting cells and to express a homologous protein to a targeted endogenous protein.

The invention showed that depending of the amount of vector used, either:
- a sustained and efficient immune response against both the homologous and the endogenous protein is induced, and completely block all the normal functions of the latter **(functional knock-out);** or,
- an efficient immune response against the vector is induced, but no or few immune response (neutralizing antibodies production) against the homologous protein is unduced allowing a long lasting expression of a transgene **(transgenesis).**

### 3.1 Concerning the functional KO

The injection of the recombinant endogenous protein is usually insufficient in its native form to induce a sustained and efficient immune response against the endogenous protein.

In the past, many attempts to do so have been tried. Among them the chemical modification of the targeted protein or the co-injection of adjuvant have been used. Numerous authors showed that induction of a specific immune response is possible but often insufficient to completely block the function of the protein. In addition multiple injection (2 to 4) are needed to enhance the affinity of the specific antibodies.

The instant invention addresses many important issues related to the efficacy of a vaccination against an endogenous protein.

The working example demonstrates that a unique injection of an adenovirus encoding an homologous protein specifically inhibits the endogenous protein. The observed effects was due to the induction of a polyclonal antibody-response, all the raised antibodies being cross reactive. The B cell hybridoma derived from mice splenocytes showed no neutralization of each clone alone, but polyclonal antibodies present in the mice sera efficiently neutralized both the endogenous and the heterologous protein.

One of the important feature of the invention is that there is no need to a high affinity binding interaction between the antibodies and the protein.

The *in vitro* neutralization assay used showed that the antibody response neutralized more efficiently the homologous than the endogenous protein.

Despite this lowest affinity, also demonstrated by the lowest binding of the antibody present in the sera against the endogenous protein, the polyclonal antibody response neutralized efficiently for up to 8 months all the in vivo activities of the targeted protein.

The importance of this results are more evident when compared to the results obtained with all the methods used to date to vaccinate against a self protein (for review see Zagury *et al.,* 2001).

These results are important for all the teams working on the generation of knock-Out mice since the usual time needed to obtain a KO mice by conventional homologous recombination techniques is more or less 24 months. Indeed, the generation of conventional KO in mice is a long and tedious process since homologous recombination is a rare event. Molecular biologists must perform numerous screenings on ES cells before to select the correctly recombined ES clone. Mice genotypings and matings are also time-consuming. Fast obtaining of functional KO for secreted and membrane proteins will be a useful tool for the characterization of functions of novel proteins.

The instant invention needs a very limited number of mice and tests for determining the effectiveness of the functional inactivation of an endogenous protein. All the necessary elements to perform such a functional KO are described in the patent and a skilled person in the art should easily use them.

A total of 6 to 8 different mice points have to be done and a single day Elisa test will confirm the occurrence of the functional inactivation. In addition if the experience have to be repeated after that a unique mice point will be enough for each repetition. These results are important for everyone in the field and our description must be put in the context of genetic KO.

### 3.2 Concerning the transgenesis

The invention focuses on antigen presenting cells (APC) because the Applicant believes that expression of the modified self protein in these cells is important for the induction of an autoreactive response. Previous published results address the question of the immunization against an erogenous protein using recombinant DNA vehicles (Ambriovic *et al*, 1997). The use of promoter which avoid expression into APC induces no immune response against the transgene encoded protein (Pastore *al*, 1999; Van Linhothout *al*, 2002).

The inventors emphasize the role of the viral dose to use for the vaccination because experimental results clearly showed that opposite effects can be observed depending on the dose used. These effects seem to be related to the inactivation of the APC by the high dose of the virus.

**3.3** The "thrombopoïetin" example used by the inventors, is just a proof a principle and not a particular case and could be repeated for every secreted or membrane protein since autoreactive immune response to numerous secreted and membrane proteins was previously described in human (For review see Zagury *al*, 2001).

The invention may be performed with any secreted or membrane protein. Induction of autoreactive immune response to numerous secreted, membrane or cellular proteins have been previously described both in human and mice (Wucherpfunnig et *al*, 1995 ; Zhao *et al,* 1998 ; Panoutsakopoulou et *al*, 2001 ; Lenin 2002). For *review see Zagury al*, 2001 ; Tanej et *al*, 2001 *;* Ermann et *al*. 2001 ; Wucherpfunnig et *al,* 2001 *;* Benoist et *al.* 2001)

Adenovirus is used in the description, also as a proof of principle and not as a particular example since it works via APC both for the immunogenic induction or the tolerigenic induction. All viruses or pathogens working in the same way (Drake et *al,* 2000; Braun et *al.* 2000; Lapointe et *al,* 2001; Havenga et *al,* 2002; Mercier et *al,* 2002; Esslinger et *al,* 2002) should also induce the same phenotype.

Since autoimmunity is described in all mammalian species, it will be easy for someone skilled in the art to use this technique to obtain a similar phenotype. However doses may need an adaptation for each of the species. The invention is well described and no undue experimentation is required for someone skilled in to the art to practice the invention.

The inventors wishes to recall that all the virologist and gene therapist using the adenovirus as a vector know that the amount of non infectious particles compared to viral particle is variable from a viral stock preparation to another one (Mittereder et *al,* 1996). Knowing that many users utilize different viral doses, both during *in vitro* or *in vivo* tests, to ensure that the desire effects is observed (Zieglet et *al,* 2002; Sung et *al*, 2002).

The results described here clearly showed that multiple doses of viral particles may be used, from 10⁹ pfu to 10¹⁰ pfu. The inactivation activity (knock-out applications) is close to 10⁹ pfu and that of tolerance (transgenesis application) is close to 10¹⁰ pfu. A narrow of viral dose between 4x10⁹ to 6x10⁹ pfu was characterized for inventor's viral preparation showing that multiple viral dose must be used to find out for each preparation the necessary dose to be use to induce functional inactivation of the endogenous protein. Scientists doing mice experiments with adenoviral vector are familiar with this practice (Zieglet et *al*, 2002; Sung et *al*, 2002).

Finally for a user a typical experience should be like this:
- three to four mutants, each one in an adenovirus construction with one of the promoters above listed. Mutation should maintain an homology of 76%, 85%, 90% and 95% for example. Other mutants in this narrow could also be used.
- 3 to 4 dosages of the virus should be used 5x10⁸, 10⁹, 2x10⁹ and 4x10⁹ for example. Other dosage in this narrow could be used but one may consider the total number of viral particle which may not exceed 10 fold. If so, doses may be adapted to be below the one we used here as an example.
- test the mice sera at 2 months by specific Elisa test as described in the patents to confirm polyclonal activation against the self protein. Elisa test could also be confirmed at 4 months before phenotypic analysis of the mice.
5 to 10 mice should be used for each point, which is largely below the necessary number of mice in the genetic KO technique.

**3.4** Concerning the variability to introduce into the cDNA sequence, the inventors showed in the working example 2.1 that 76 % identity induces this strong cross reactive polyclonal response against both the self protein and the homologous protein. This effect is specific since no neutralization activity was observed with a protein, the erythropoeitin, having around 50% homology with the targeted protein. This was demonstrated by the normal hematocrit, a marker of the erythropoietin level, of all tested mice.

It is already known that pathogens, mainly viruses, can induces in some cases autoimmunity by so called molecular mimicry mechanisms (Wucherpfunnig et *al*, 1995 ; Zhao *et al,* 1998 *;* Panoutsakopoulou et *al*, 2001 ; Levin 2002). For *review see Zagury al.* 2001 *;* Tanej et *al*, 2001 *;* Ermann et *al*, 2001 ; Wucherpfunnig et *al*, 2001 *;* Benoist et *al*, 2001).

Variability may concern a unique amino acid change to a greater variability. This is also to determine for each protein and will be evident for everyone in the field who tend to obtain the same phenotype than the one the inventors describe. Variability from 76% to 99.9% identity could be used to obtain the functional inactivation of an endogenous protein phenotype. Three to four modifications in this narrow may be sufficient.

### REFERENCES CITED

Abina et al. (1998) J. Immunol. 160:4481-4489
Alexander et al. (1996) Blood 87:2162-2170
Ambriovic et al, (1997)Virology 238:327-335
Armentano et al. (1993) Human Gene Therapy 6:1343
Armentano et al. (1995) Hum. Gene Ther. 6:1343-1353
Armentano et al., (1997) J. Virol. 71:2408
Ausubel et al. (1995) Current Protocols in Molecular Biology, Eds., John Wiley & Sons, Inc. New York
Benoist et al, (2001)Nature Immunol. 2:797-800
Berkner (1992) Curr. Top. Micro. Immunol., 158:39-66
Braun et al, (2000)Gene Ther 7: 1147-1457
Carver-Moore et al. (1996) Blood 88:803-808
Drake et al, (2000) J. Virol 74: 1093-4101
Ermann et al, (2001)Nature Imunol 2:759-761
Esslinger et al, (2002) Hum Gene Ther 13: 1091-1100
Fang et al. (1995) Hum. Gene Ther. 6:1039-1044
Fisher et al. (1996)) Virology, 217:11-22
Gorziglia et al. (1996) J. Virol. 70:4173-4178
Gough et al. (1985) EMBO J. 3:645-653
Graham (1977) **36**:59-72
Guo et al. (1996) Gene Therapy 3:801-802
Havenga et al, (2002) J. Virol 76: 4612-4620
Ho et al. (1989) Gene 77:51-59
Hostomsky et al. (1989) Biochem. Biophys. Res. Comm 161:1056-1063)
Imler et al. (1996) Gene Therapy 3:75-84
Jolly (1994) Cancer Gene Therapy, 1:51-64
Kay et al. (1995) Nature Genetics, 11:191-197
Kaplan et al. (1997) Human Gene Therapy 8:45-56
Kochanek et al. (1996) Proc. Natl. Acad. Sci. USA 93:5731-5736
Lapointe et al, (2001) J. Immunol 167 :4758-4764
Leung et al. (1989) Technique 1:11-15
Levin et al, (2002) Nature Med 8: 509-513
Lieber et al. (1996) J. Virol. 70:8944-8960
Maione et al. (2001) Proc. Natl. Acad. Sci. USA 98: 5986-91.
Mayers et al.(1985) Science 229:242)
Mercier et al, (2002) J. Virol 76: 2899-2911
Mittereder et al, (1996) J. Virol 70: 7498-7509
Neddleman et Wunsch (1970) J. Mol. Biol. 48 : 443
Pearson et Lipman (1988) Proc. Natl. Acad. Sci. USA 85:2444
Remington's Pharmaceutical Sciences (1985) 17th ed., Mack Publishing Co., Easton, Pa.
Panoutsakopoulou et al, (2001) Immunity 15:137-147
Pastore et al, (1999) Hum Gene Ther 10:1773-1781
Sambrook et al. (1989) Molecular cloning : A Laboratory Manual 2nd Edition - Cold Spring Harbor Laboratory Press - Cold Spring Harbor, NY, USA
Smith et Waterman (1981) Ad. App. Math. 2:482
Stradford-Perricaudet et al. (1990) Hum. Gene Ther. 3:241-256
Sung et al, (2002) Hum gene Ther 13:731-743
Taneja et al, (2001) Nature Immunol 2:791-784
Tripathy et al. (1996) Nature Med. 2:545-550
Van Linhothout et al, (2002) Hum gene Ther 13: 829-840
Wendling et al. (1994) Nature 369:571-574
Wucherpfunnig et al, (1995)Cell 80:695-705
Wucherpfunnig et al, (2001) J Clin Invest 108:1097-1104
Yang et al. (1994a) Immunity 1:433-442
Yang et al. (1994) Nature Genetics, 7:362-369
Yang et al. (1994) Proc. Natl. Acad. Sci. USA 91:4407-4411
Yang et al. (1996) Gene Ther. 3:137-144
Yang et al. (1996) J. Virol. 70:7202
Zagury et al., (2001)Proc Nat1 Sci USA 98:8024-8029
Zhao et al, (1998)Science 279:1344-1347
Ziegler et al, (2002) Hum Gene Ther 13:935-945
Zsellenger et al. (1995) Hum. Gene Ther. 6:457-467

## Claims

1. Method of inhibiting in a mammal formation of neutralizing antibodies directed against an heterologous protein comprising the step of co-administering to said mammal, an agent in an amount sufficient to deplete or inhibit at least some antigen presenting cells of said mammal, and said heterologous protein and/or a nucleic acid sequence encoding said heterologous protein, said agent being administered prior or simultaneously to said heterologous protein and/or a nucleic acid sequence, thereby inhibiting the production of neutralizing antibodies against said heterologous protein.

2. Method according to claim 1, wherein said agent is selected among viruses, liposomes, antibodies, parasites, bacteriae, funguses and or fragments thereof, and nucleic acid sequence encoding said heterologous protein.

3. Method according to claim 2, wherein said virus is selected among adenovirus, adenovirus associated virus, retrovirus, pox virus, vaccinia virus, or fragments thereof.

4. Method according to claim 3, wherein said adenovirus is selected among wild type human adenovirus and recombinant adenovirus, or a fragment thereof.

5. Method according to claims 1 to 4, wherein said antigen presenting cells are antigen presenting cells located in liver of said mammal.

6. Method according to claim 2, wherein said agent is administered prior said heterologous protein and/or said nucleic acid sequence encoding said heterologous protein.

7. Method according to claim 2 wherein said agent is administered simultaneously to said heterologous protein and/or said nucleic acid sequence encoding said heterologous protein.

8. Method according to claim 7, wherein said agent and said nucleic acid sequence encoding said heterologous protein are simulatenously co-administered as a recombinant virus, the genome of which comprising at least nucleic acid sequence encoding said heterologous protein.

9. Method according to claim 8, wherein the genome of said recombinant virus comprises at least regulation sequences necessary to direct the expression of said heterologous protein in at least one antigen presenting cell of said mammal.

10. Method according to claim 9, wherein said regulation sequences comprises promoter sequences selected among cytomegalovirus early promoter (CMV IEP), Rous sarcoma virus long terminal repeat promoter (RSV LTR), myeloproliferative sarcoma virus long terminal repeat (MPSV LTR), simian virus 40 early promoter (SV40 IEP), major late promoter of the adenovirus.

11. Method according to claims 1 to 10, further comprising the step of administering to said mammal additional agent to enhance the depletion and/or the inhibition of at least some antigen presenting cells of said mammal.

12. Method of inhibiting in a mammal formation of neutralizing antibodies directed against an heterologous protein comprising the step of administering to said mammal a recombinant adenovirus, the genome of which comprising at least a nucleic acid sequence encoding said heterologous protein and regulation sequences, in an amount sufficient to deplete or inhibit at least some antigen presenting cells of said mammal, thereby inhibiting the production of neutralizing antibodies against said heterologous protein.

13. Method according to claim 12, further comprising the step of administering to said mammal additional adenovirus or a fragment thereof, the genome of which not expressing said heterologous protein, thereby enhancing the amount of adenoviruses to deplete or inhibit at least some antigen presenting cells of said mammal.

14. Method according to claims 12 to 13, wherein said mammal is a mouse and wherein the amount of adenovirus particules administered to deplete or inhibits at least some antigen presenting cells of said mouse is equal or greater to 4.10¹⁰ particles, said particles comprising optionally said additional adenovirus.

15. Method according to claim 14, wherein the amount of adenovirus particules administered to deplete or inhibits at least some antigen presenting cells of said mouse is equal or greater to 6.10¹⁰ particles.

16. Method according to claims 14 and 15, wherein the amount of said recombinant adenovirus able to form plaque, is equal or greater to 4.10⁹ pfu/mouse.

17. Method of producing transgenic mammal expressing an heterologous protein said method comprising the step of inhibiting in said mammal formation of neutralizing antibodies directed against said heterologous protein by the use of the method of claims 1 to 16 thereby allowing a long-lasting expression of said heterologous protein.

18. Method according to claim 17, wherein the mammal is selected among mouse, rat, rabbit, cow, pig, goat, sheep.

19. Method for reducing an anti-heterologous protein immune response in a mammal, including human, subject to the administration of said heterologous protein and/or nucleic acid sequence encoding said heterologous protein, said method comprising the step of inhibiting in said mammal formation of neutralizing antibodies directed against said heterologous protein by the method of claims 1 to 16.

20. Method according to claim 19, wherein said method is a step of a gene therapy protocol for the treatment of human afflicted with a disease selected among inheritated or acquired genetic diseases, infectious diseases, inflammatory diseases, autoimmune diseases, cancers, and the associated syndromes thereof.

21. Method for the therapy of a mammal, including humans, afflicted with a disease **characterized by** the altered expression of an endogenous protein, said method comprising the step of administering to said mammal said protein and/or nucleic acid sequence encoding said protein, and simultaneously or previously, the step of inhibiting in said mammal formation of neutralizing antibodies directed against said protein by the method of claims 1 to 16.

22. Method according to claims 20 and 21, further comprising the step of co-administering simultaneously, separately or sequentially, to said mammal at least one immune modulators selected among cyclosporin, cyclophosphamide, FK506, desoxyspergualine, interleukin-4, interleukin-12, interferon-gamma, anti-CD4 monoclonal antibody, anti-CD8 monoclonal antibody, anti-LFA1 monoclonal antibody, antibody directed against CD40 ligand or CTLA4Ig.

23. Method of modulating in a mammal formation of neutralizing antibodies directed against an heterologous protein, said method comprising the steps of:
(i) Optionally, co-administering to a first mammal, at least one agent and said heterologous protein and/or a nucleic acid sequence encoding said heterologous protein, said agent being administered simultaneously, sequentially or separately with said heterologous protein and/or nucleic acid sequence, and determining at least one amount of said heterologous protein and said agent, sufficient to trigger an immune response against said heterologous protein by said first mammal; optionally, re-performing step (i) until said amount is determined;
(ii) co-administering to a second mammal said heterologous protein and/or a nucleic acid sequence encoding said heterologous protein, in an amount sufficient to trigger an immune response against said heterologous protein, as determined at step (i) and prior or simultaneously, said agent, in an amount greater that the one determined at step (i) and sufficient to trigger an immune response against said agent and sufficient to deplete or inhibit at least some antigen presenting cells of said mammal, and determining for said second mammal at least one amount of said agent that reduces and/or suppresses the anti-heterologous protein immune response in said mammal; re-performing step (ii) until said amount is determined; and
wherein,
(a) when one administers to a third mammal, said agent in an amount equal or greater than the one determined at step (i) but lesser than the one determined at step (ii), said mammal produces neutralizing antibodies against said heterologous protein and optionnally against said agent; and
(b) when one administers to said mammal said agent in an amount equal or greater than the one determined at step (ii), said mammal produces neutralizing antibodies against said agent but produces no or few neutralizing antibodies against said heterologous protein.

24. Method according to claim 23, wherein an additional agent is further administered to said mammal in step (i) and (ii).

25. Method according to claims 23 and 24, wherein the amount of said agent of step (ii) is at least twice the amount of said agent determined at step (i).

26. Method according to claims 23, 24, 25, wherein said mammal is a mouse and said agent is an adenovirus, and wherein said agent and said nucleic acid sequence encoding said heterologous protein are simulatenously co-administered as a recombinant adenovirus, the genome of which comprising at least said nucleic acid sequence encoding said heterologous protein and wherein :
- the amount of said recombinant adenovirus particles of step (i) that triggers an immune response towards said heterologous protein in said mouse without depleting or inhibiting at least some antigen presenting cell of said mouse is below 4.10¹⁰ particles, and/or the amount of said adenovirus particles able to form plaque is below 4.10⁹ pfu/mouse; and
- the amount of said recombinant adenovirus particles of step (ii) that reduces or suppresses the anti-heterologous protein immune response in said mouse is at least equal or greater than 4.10¹⁰ particles and/or the amount of said adenovirus particles able to form plaque is equal or greater than 4.10⁹ pfu/mouse.

27. Use of a method according to claim 23 of inhibiting in a mammal formation of neutralizing antibodies directed against an heterologous protein, said method comprising the step of co-administering to said mammal, said heterologous protein and/or a nucleic acid sequence encoding said heterologous protein and prior or simultaneously said agent in an amount equal or greater than the one determined at step (ii).

28. Use of a method according to claim 23 of triggering in a mammal formation of neutralizing antibodies directed against an heterologous protein, said method comprising the step of co-administering simultaneously, separately or sequentially to said mammal said heterologous protein and/or a nucleic acid sequence encoding said heterologous protein, and said agent in an amount and/or concentration equal or greater than the one determined at step (i) but lesser than the one determined at step (ii).

29. Method for the therapy of a mammal affected by a disease wherein at least one endogenous protein is involved in said disease ethiology, said method comprising the step of inhibiting the biological functions of said endogenous protein by enhancing the production of neutralizing antibodies against said protein by use the method according to claim 23.

30. Method according to claim 29, wherein said disease is chosen among auto-immune diseases, inflammatory diseases, cancers, viral infections, bacterial infections, parasites infections, funguses infections.

31. Use of a method according to claim 28 to produce a mammal with a functional inactivation of at least one endogenous protein, said method comprising the step of administering to a mammal in a simultaneous, separate or sequential manner at least one agent and an heterologous protein and/or a nucleic acid sequence encoding for said heterologous protein, said nucleic acid sequence being expressed in at least one cell of said mammal, wherein said heterologous protein being substantially identical to said endogenous protein wherein the amount of said heterologous protein, optionally of said agent, that is administered to said mammal is the one determined in step (i), thereby the amount of anti-heterologous neutralizing antibodies produced by said mammal being sufficient to alter the biological activity of said heterologous protein and /or of said endogenous protein.

32. Use according to claim 31, wherein said heterologous protein is at least 50% identical to the endogenous protein.

33. Use according to claim 32, wherein said heterologous protein is a protein selected among animal species, including humans, homologous to said endogenous protein of said mammal.

34. Use according to claim 33, wherein said heterologous protein is mutated in order to enhance its immunogenicity.

35. Method of producing an animal with a functional inactivation phenotype by inactivating at least one endogenous protein, said method comprising the step of triggering in said mammal formation of neutralizing antibodies directed against an heterologous protein being substantially identical to said endogenous protein, said method comprising the step of co-administering to said mammal in a simultaneous, separate or sequential manner, at least one agent and said heterologous protein and/or a nucleic acid sequence encoding for said heterologous protein, said nucleic acid sequence being expressed in at least one cell of said mammal, wherein the amount of said heterologous protein, optionally of said agent, is at least sufficient to trigger an immune response against said heterologous protein and the amount of said agent is not sufficient to deplete or inhibite at least some antigen presenting cells of said mammal.

36. Mammal obtained by the method of claim 35.

37. Mammal of claim 36, wherein said mammal is a mouse, rat, rabbit.

38. Use of a mammal according to claims 36 and 37 to perform biological, physiological, biochemical, molecular studies and analysis of the function of said heterologous and/or homologous protein.

39. Use of a mammal according to claims 36 and 37 to perform drug screening.

40. Use of a mammal according to claims 36 and 37 to isolate spleen cells from said mammal that expresses antibody directed against said heterologous an/or endogenous protein to make hybridoma(s).

41. Use of biological fluid of the mammal according to claims 26 and 27 to prepare serum and/or polyclonal antibodies.

42. Method to produce vaccine for a mammal, against an heterologous protein, said method comprising the step of triggering in said mammal formation of neutralizing antibodies directed against said heterologous protein, by using the method according to claim 23.

43. Method according to claims 1 to 26 and 33, use of a method according to claim 27 to 32, wherein said heterologous protein or a fragment thereof is selected among the proteins that are presented by class I major histocompatibility molecule (CMH I), a class II major histocompatibility molecule (CMH II), or a combination of a class I major histocompatibility molecule and a class II major histocompatibility molecule.

44. Method according to claim 43, wherein said heterologous protein is chosen among secreted proteins, membranes proteins, receptors, intracellular proteins, nuclear proteins.

45. Method according to claim 44, wherein said secreted protein is selected among neuromediators, hormones, interleukines, lymphokines, interferons, chemokines, growth factors.

46. Method according to claims 1 to 26 and 33, wherein the mammal is chosen among mouse, rat, rabbit, hamster, Chinese pig, cow, pig, goat, sheep, horse, primate.

47. Method according to claims 1 to 26 and 33, wherein the administration of said agent and said heterologous protein and/or nucleic acid sequence encoding said heterologous protein is performed via a technique chosen among intravenous injection, intravaginal injection, intrarectal injection, intramuscular injection, intradermic injection,

48. Method according to claim 47, wherein said intravenous injection is selected among retro-orbital sinus injection, tail injection, hepatic injection, femoral or jugular injection.
